(19) Europäisches Patentamt
European Patent Office
Office européen des brevets

(11) EP 0 859 058 A2

(12) EUROPÄISCHE PATENTANMELDUNG

(43) Veröffentlichungstag:
19.08.1998 Patentblatt 1998/34

(51) Int. Cl.⁶: **C12N 15/85**, C12N 15/12,
C12N 15/52, C12N 9/00,
C12N 9/64, C07K 14/47,
A61K 48/00

(21) Anmeldenummer: 98100632.3

(22) Anmeldetag: 15.01.1998

(84) Benannte Vertragsstaaten:
AT BE CH DE DK ES FI FR GB GR IE IT LI LU MC
NL PT SE

(30) Priorität: 16.01.1997 DE 19701141

(71) Anmelder:
HOECHST AKTIENGESELLSCHAFT
65929 Frankfurt am Main (DE)

(72) Erfinder:
• Heidtmann, Hans Heinrich, Dr.
27568 Bremerhahven (DE)

• Müller, Rolf, Prof. Dr.
35037 Marburg (DE)
• Sedlacek, Hans-Harald, Prof. Dr.
35041 Marburg (DE)

(74) Vertreter:
Bösl, Raphael, Dr. rer. nat., Dipl.-Ing. et al
Patent- und Rechtsanwälte
Bardehle . Pagenberg . Dost . Altenburg .
Frohwitter . Geissler,
Galileiplatz 1
81679 München (DE)

(54) **Nukleinsäurekonstrukt zur Expression von durch Proteasen aktivierbare Wirksubstanzen, sowie Herstellung und Verwendung**

(57) Die Erfindung bezieht sich auf ein Nukleinsäurekonstrukt, zur Expression einer Wirksubstanz, die durch ein von Säugerzellen freigesetztes Enzym aktiviert wird, welches folgende Komponenten enthält:

a) mindestens ein Promotorelement,
b) mindestens eine DNA-Sequenz, die für einen Wirkstoff (Protein B) kodiert,
c) mindestens eine DNA-Sequenz, die für eine Aminosäuresequenz (Teilstruktur C) kodiert, welche durch ein von einer Säugerzelle freigesetztes Enzym spezifisch spaltbar ist, und

d) mindestens eine DNA-Sequenz, die für ein Peptid oder Protein (Teilstruktur D) kodiert, welches über die spaltbare Aminosäuresequenz (Teilstruktur C) an den Wirkstoff (Protein B) gebunden ist und die Wirksamkeit des Wirkstoffes (Protein B) hemmt sowie

die Verwendung des Nukleinsäurekonstruktes zur Herstellung eines Heilmittels zur Behandlung von Krankheiten.

Figur 1

vom Strukturgen kodiertes Protein BCD

Printed by Xerox (UK) Business Services
2.16.3/3.4

**Beschreibung**

Die vorliegende Erfindung bezieht sich auf ein Nukleinsäurekonstrukt zur Expression von durch Proteasen aktivierbare Wirksubstanzen sowie dessen Herstellung und Verwendung.

Ähnlich wie entzündete Bereiche unterscheiden sich Tumoren von dem umgebenden Normalgewebe durch eine erhebliche Zunahme der Bildung und Sekretion von Proteasen [Schmitt et al., Fibrinol. 6, 3 (1992), Cottam et al., Int. J. Oncol. 2, 861 (1993), Tryggvason et al., Breast Cancer Res. And Treatm. 24, 209 (1993), Leto et al., Anticancer Res. 12, 235 (1992), Hart, Fibrinol. 6, 11 (1992), Albini et al., J. Natl. Cancer Inst. 83, 735 (1991)]. Zu diesen gehören beispielsweise Plasminogenaktivatoren, Cathepsine und Matrix-Metalloproteinasen.

Eine wesentliche Funktion dieser Tumorproteasen ist die Auflösung der extrazellulären Matrix für die Einwanderung und das infiltrative Wachstum der Tumorzellen in Normalgewebe. Zugleich stellen diese Proteasen einen Schutz des Tumors vor den körpereigenen Abwehrmechanismen dar, in dem die für die Abwehr notwendigen Wirkstoffe durch die vom Tumor gebildeten Proteasen gespalten und damit inaktiviert werden. So werden beispielsweise Antikörper, Zytokine und Wachstumsfaktoren, Komplementfaktoren, Gerinnungsfaktoren und Mediatoren durch Tumorproteasen inaktiviert.

In der Vergangenheit war es daher das Ziel, durch Hemmung der Tumorzellproteasen das infiltrative Wachstum und Metastasenwachstum von Tumoren sowie die Inaktivierung von körpereigenen Abwehrmechanismen zu inhibieren [Hocman, Int. J. Biochem. 24, 1365 (1992), Troll et al., JNCI 73, 1245 (1984), Ray et al., Eur. Respir. 7, 2062 (1994), Koop et al., Cancer Res. 54, 4791 (1994), Chiriri et al., Int. J. Cancer 58, 460 (1994), Denhardt et al., 59, 329 (1993), Melchiori et al., Cancer Res. 52, 2353 (1992)]. Aus insbesondere stöchiometrischen wie auch pharmakokinetischen Gründen, ist die Hemmung der Tumorzellproteasen bislang nur wenig erfolgreich gewesen.

Es wurde daher versucht, die Tumorzellproteasen für eine Aktivierung von bakteriellen Toxinen wie beispielsweise des α-Hämolysins von Staphylococcus aureus zu nutzen [Panchal et al., Nature Biotechn. 14, 852 (1996)]. Hierzu wurde eine Aminosäuresequenz XX-Arg-X in die Position 129 bis 132 des α-Hamolysins eingefügt und so inaktive Mutanten erzeugt, welche erst durch Tumorproteasen, wie z.B. Cathepsin B, gespalten und dadurch aktiviert werden.

Auf der Basis dieser Ergebnisse wurden Proimmunolysine vorgeschlagen [Panchal et al., Nature Biotechn. 14, 852 (1996)], welche aus einem Antikörper bestehen, der an ein durch Tumorproteasen aktivierbares α-Hämolysin von Staphylococcus aureus oder ein Equinatoxin II von der Seeanemone gekoppelt ist, wobei der Antikörper die Zielzellspezifität des Kopplungsproduktes bestimmt.

Das vorgeschlagene Konzept hat jedoch für eine Tumortherapie folgende Nachteile:

Zum einen wurden xenogene körperfremde Lysine bzw. Toxine ausgewählt, welche für den Wirtsorganismus (Patienten) immunogen sind und dadurch im Wirtsorganismus eine Immunreaktion auslösen, welche das Antikörpertoxinkonjugat neutralisieren und inaktivieren. Zum anderen ist von tumorspezifischen Antikörpern wie auch Immuntoxinen bekannt [Sedlacek et al., Antibodies as Carriers of Cytotoxicity, Contrib. to Oncol. 43, Karger Verlag, München, 1992), daß sie aufgrund ihrer Molekülgröße wie auch der rheologischen Bedingungen am Tumor nur in einer sehr geringen Menge (0,01 - 0,001% des gegebenen Antikörpers bzw. Immuntoxins/g Tumor) am Tumor lokalisieren und diesen nur unvollständig penetrieren, so daß nicht alle Tumorzellen oder nur ein geringer Teil der Tumorzellen eines Tumors abgetötet werden können. Wiederum ist die Expression von Tumorantigenen, gegen welche der Antikörper gerichtet ist, zwischen den einzelnen Tumorzellen meist unterschiedlich und die variablen Antigen-negativen Tumorzellen entziehen sich leicht dem Angriff der Antikörper bzw. Immuntoxine. Von den Tumorzellen abgesonderte Antigene neutralisieren zudem die Antikörper in der Peripherie des Tumors (Sedlacek et al., Monoclonal Antibodies in Tumor Therapy, Contrib. to Oncol., Karger Verlag, 1988).

Folglich besteht weiterhin ein großer Bedarf nach einer zielzellspezifischen Therapie von Tumoren und Entzündungen. Aufgabe der vorliegenden Erfindung ist daher, einen Wirkstoff gegen Tumore und Entzündungen bereitzustellen, der die genannten Nachteile nicht aufweist. Die vorliegende Erfindung bezieht sich daher auf eine neue Technologie, welche die Ausschüttung von Enzymen in Tumoren oder Entzündungsgebieten für die lokale Freisetzung von Wirkstoffen nutzt, deren inaktive Vorstufen in Tumorzellen, tumorassoziierten Zellen oder Entzündungszellen exprimiert werden.

Ein Gegenstand der vorliegenden Erfindung ist daher ein Nukleinsäurekonstrukt zur Expression einer Wirksubstanz, die durch ein von Säugerzellen freigesetztes Enzym aktiviert wird, das folgende Komponenten enthält:

a) mindestens ein Promotorelement,
b) mindestens eine DNA-Sequenz, die für einen Wirkstoff (Protein B) kodiert,
c) mindestens eine DNA-Sequenz, die für eine Aminosäuresequenz (Teilstruktur C) kodiert, welche durch ein von einer Säugerzelle freigesetztes Enzym spezifisch spaltbar ist, und
d) mindestens eine DNA-Sequenz, die für ein Peptid oder Protein (Teilstruktur D) kodiert, welches über die spaltbare Aminosäuresequenz (Teilstruktur C) an den Wirkstoff (Protein B) gebunden ist und die Wirksamkeit des Wirkstoffes (Protein B) hemmt.

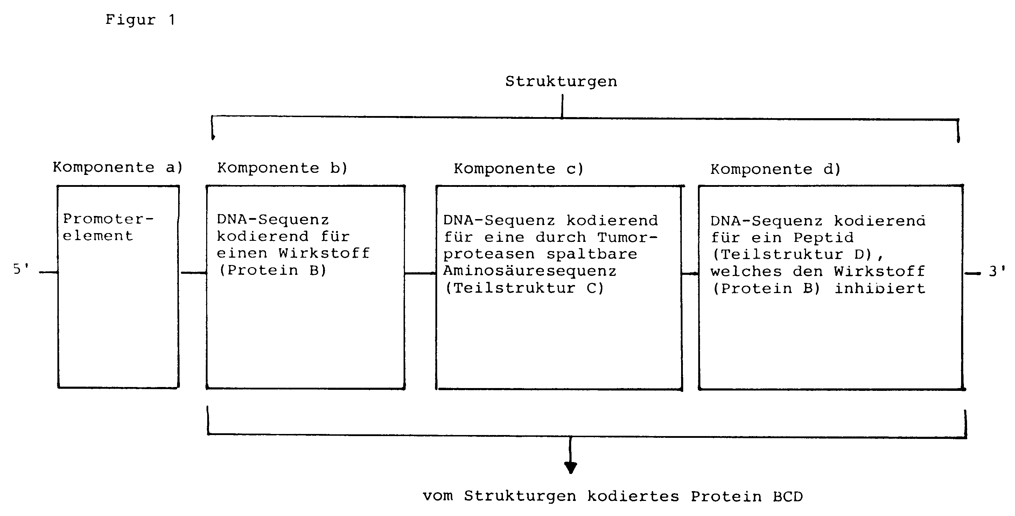

Die einzelnen Komponenten können in ihrer einfachsten Form beispielsweise wie in Figur 1 gezeigt angeordnet sein. Die Expression eines Proteins BCD, kodiert von den Komponenten b), c) und d) wird hierbei durch eine Aktivierung der Promotorsequenz [Komponente a)] induziert. Die Aminosäuresequenz C des Expressionsprodukts wird anschließend durch zelluläre Enzyme, z.B. Proteasen gespalten, wodurch das Protein B freigesetzt wird, welches den aktiven Wirkstoff darstellt. Proteasen oder Enzyme bedeuten im Sinne der vorliegenden Erfindung ein oder mehrere Proteasen oder Enzyme.

In einer weiteren Ausführungsform ist das genannte Enzym eine Protease, insbesondere ein Prostata-spezifisches Antigen, ein Plasminogenaktivator, ein Cathepsin oder eine Matrix-Metalloproteinase. Die genannten Säugerzellen sind vorzugsweise Tumorzellen, Leukämiezellen, Endothelzellen, Makrophagen, Lymphozyten, Muskelzellen, Epithelzellen, Gliazellen, Synovialzellen oder virusinfizierte Zellen.

Enzyme werden in einem Organismus bevorzugt freigesetzt von Tumoren und Tumorzellen, wie auch von Zellen, welche an einem Entzündungsgeschehen beteiligt sind [Barrett et al., Mammalian Proteases, Academic Press, London 1980; Sedlacek und Möröy, Immune Reactions, Springer Verlag, 1995)].

Gemäß der vorliegenden Erfindung ist die Komponente c) folglich derart ausgewählt, daß das exprimierte Protein, z.B. BCD, bevorzugt in seiner Teilstruktur C von Proteasen, die in Tumoren gebildet werden oder von Tumorzellen oder Entzündungszellen sezerniert werden, gespalten wird. Beispielsweise gehören hierzu Plasminogenaktivatoren, wie Plasminogenaktivator vom Urokinasetyp oder der Gewebe-Plasminogenaktivator; Cathepsine, wie das Cathepsin B, das Cathepsin D, das Cathepsin L, das Cathepsin E oder das Cathepsin H bzw. deren Vorstufen (ProCathepsine); Matrix-Metalloproteinasen (MMP), wie Collagenasen beispielsweise der Gruppen I, II, III, IV oder V; Stromelysin 1, - 2 oder - 3; Metrilysine; Gelatinasen, wie Gelatinase A (MMP-2), Progelatinase B (MMP-9) und A [Pappot et al., Lung Cancer 12, 1 (1995), Schmitt et al., Fibronolysis 614, 3 (1992), Monsky et al., Cancer Biol. 4, 251 (1993), Rochefort et al., Medecine/Sciences 7, 30 (1991), Kao et al., 46, 1349 (1986), Fridman et al., Cancer Res. 55, 2548 (1995), Ray et al., Eur. Respir. J. 7, 2062 (1994), Cottam et al., Int. J. Oncol. 2, 861 (1993), Tryggvason et al., Breast Cancer Res. and Treatm. 24, 209 (1993)]; Tumorzelloberflächenproteasen [surface expressed proteases = Seprase; Monsky et al., Cancer Res. 54, 5702 (1994)]; Elastase [Kao et al., Cancer Res. 46, 1355 (1986)]; Prostata-spezifisches Antigen [Lundwall, Biochem. Biophys. Res. Commun. 161, 1151 (1989), Riegman et al., Biochem. Biophys. Res. Commun 159, 95 (1989)] oder pankreatische Trypsinogene [Miszuk-Jamska et al., FEBS Lett. 294, 175 (1991)].

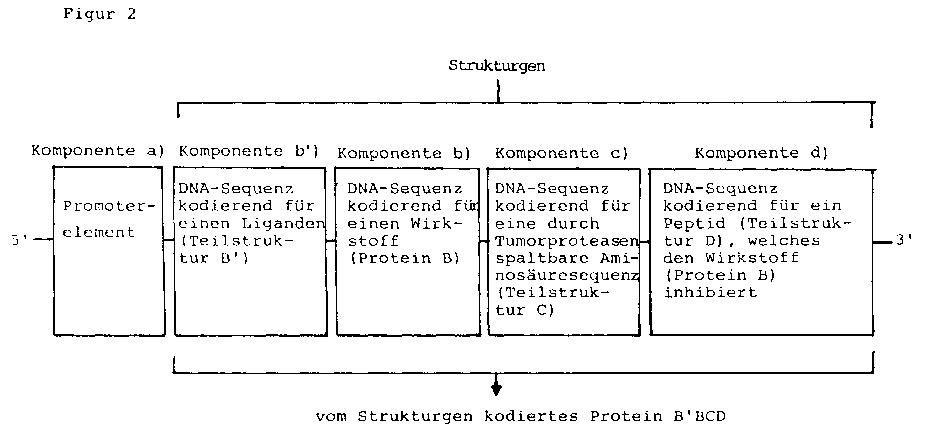

Entsprechend einer weiteren Ausführungsform der vorliegenden Erfindung kann die Nukleotidsequenz für die Komponente b) um eine Komponente b') erweitert werden. Diese Komponente b') kodiert für einen Liganden (Teilstruktur B'), welcher den Wirkstoff an eine Zielstruktur binden kann. Die Komponente b') ist beispielsweise wie in Figur 2 gezeigt angeordnet. Die Expression des Nukleinsäurekonstruktes entsprechend Figur 2 führt zu einem Protein B'BCD, welches über den Liganden (Teilstruktur B') an eine Zielstruktur bindet. Anschließend wird die Teilstruktur C durch zelluläre Proteasen gespalten, so daß der aktive Wirkstoff Protein B'B freigesetzt wird.

In einer besonderen Ausführungsform sind das genannte Protein B und die Teilstruktur D Teile der natürlichen Vorstufen von Proteinwirkstoffen, wobei die natürliche Spaltsequenz, welche die Teilstrukturen B und D verbindet, durch die Teilstruktur C ersetzt worden ist; insbesondere ist die genannte Teilstruktur D de Teilstruktur einer natürlichen Vorstufe eines Proteinwirkstoffes.

Die erfindungsgemäßen Nukleinsäurekonstrukte bestehen vorzugsweise aus DNS. Unter dem Begriff "Nukleinsäurekonstrukte" werden künstliche Gebilde aus Nukleinsäure verstanden, die in den Zielzellen transkribiert werden können. Sie sind bevorzugt in einen Vektor eingefügt, wobei Plasmidvektoren oder virale Vektoren besonders bevorzugt sind.

Durch die erfindungsgemäßen Nukleinsäurekonstrukte wird ein Strukturgen [Komponenten b) + c) + d) oder b') + b) + c) +d) ] je nach Wahl des Promotorelements [Komponente a)] entweder unspezifisch, zellspezifisch, virusspezifisch, unter bestimmten metabolischen Bedingungen, zellzyklusspezifisch oder in Anwesenheit von Tetrazyklin exprimiert. Es können auch mindestens zwei gleiche oder unterschiedliche Promotorelemente zusammen kombiniert werden für eine je nach Wahl dieser Promotorelemente eingeschränkte Expression des Strukturgens. Die Komponente a) wird bevorzugt in Endothelzellen, in Zellen benachbart zu aktivierten Endothelzellen, in Muskelzellen, in Leukämiezellen, in Tumorzellen, in Gliazellen, in Lymphozyten, in Makrophagen und/oder in Synovialzellen aktiviert.

Die Teilstruktur B (Protein B) des von dem erfindungsgemäßen Strukturgen kodierten Proteins stellt den eigentlichen erfindungsgemäßen Wirkstoff dar, der durch Spaltung der Teilstruktur C freigesetzt oder aktiviert und damit aus dem gehemmten Zustand, z.B. als Protein BCD bzw. als Protein B'BCD, in den aktiven Zustand, z.B. als Protein B bzw. als Protein B'B, überführt wird.

Dieser Wirkstoff kann entsprechend der Erfindung ein Enzym sein, welches eine biologische Aktivierungskaskade aktiviert oder inhibiert und/oder aktiver Bestandteil dieser Kaskade ist. Derartige biologische Aktivierungskaskaden sind beispielsweise das Gerinnungssystem, welches aktiviert oder inhibiert werden kann, die Fibrinolyse, welche vorzugsweise aktiviert wird, das Komplementsystem, welches ebenso vorzugsweise aktiviert wird, oder das Kininsystem, welches auch vorzugsweise aktiviert wird. Er kann auch ein Enzym sein, welches die inaktive Vorstufe einer pharmakologischen Substanz in die aktive Substanz überführt oder das selbst eine pharmakologisch aktive Substanz darstellt.

Besonders bevorzugt ist ein Wirkstoff (Protein B), der einen Gerinnungsfaktor darstellt, ausgewählt aus Thrombin, Faktor Va, Faktor VIIa, Faktor IXa, Faktor Xa, TF gerinnungsaktive Fragmente oder Faktor XIIa; Thrombin, mutiert im Bereich der Spaltstelle Arg-Thr (Aminosäureposition 327/328); ein fibrinolytisches Protein ausgewählt aus Urokinase, tPA oder funktionellen Hybriden hiervon; einen Komplementfaktor ausgewählt aus CVF, C3b oder funktionellen Spaltprodukten hiervon; ein antithrombotisches Protein ausgewählt aus Protein C, C-1S-Inhibitor, $\alpha$1-Antitrypsin, Hirudin, AT-III, TFPI, PAI-1, PAI-2 oder PAI-3; ein Kallikrein; ein zytostatisches, zytotoxisches oder entzündungserregendes Protein; ein antiangiogenetisches Protein; ein immunmodulierendes Protein; ein antientzündlichwirkendes Protein; ein Schäden des Nervensystems behebendes Protein; ein die neurotoxische Wirkung von TNF$\alpha$ inhibierendes oder neutralisierendes Protein; ein die Angiogenese stimulierendes Protein; ein den Blutdruck senkendes Protein; ein antivirales Protein; ein Cytokin; ein Interferon; einen Tumornekrosefaktor; Oncostatin M oder LIF; einen Cytokinrezeptor; den zellexternen Teil eines Cytokinrezeptors; einen Cytokinantagonisten; einen Wachstumsfaktor einen Wachstumsfaktorrezeptor; den zellexternen Teil eines Wachstumsfaktorrezeptors; ein Chemokin; Angiostatin; Platelet factor 4; TIMP-1, -2 oder -3; eine Nitroreduktase; eine $\beta$-Glucuronidase; eine Carboxypeptidase; eine $\beta$-Laktamase; eine Cytosindeaminase; eine Katalase; eine Peroxidase; eine Phosphatase; eine Oxidase; Kallikrein oder eine Endothelzellnitritoxidsynthase.

Die Teilstruktur B' des von dem erfindungsgemäßen Strukturgen kodierten Proteins stellt den erfindungsgemäßen Liganden für die Bindung des Wirkstoffes (Protein B) an eine Zielstruktur dar. Eine bevorzugte Zielstruktur ist die Oberfläche von Zellen, vorzugsweise ein Zellmembranrezeptor, ein Zellmembranantigen, ein zellmembranständiges Adhäsionsmolekül oder die extrazelluläre Matrix, wie beispielsweise von Endothelzellen, insbesondere von aktivierten oder proliferierenden Endothelzellen, Tumorzellen, Muskelzellen, insbesondere von glatten Muskelzellen, Fibroblasten, Makrophagen, Lymphozyten, Leberzellen, Nierenzellen, Synovialzellen, Entzündungszellen, mit Viren infizierte Zellen, Bronchialepithelzellen, Gliazellen, Leukämiezellen oder Zellen anderer Gewebe und Organe. Eine besonders bevorzugte Zielstruktur ist die Oberfläche von aktivierten und/oder proliferierenden Endothelzellen.

Eine weitere bevorzugte Zielstruktur sind Komponenten der extrazellulären Matrix, wie beispielsweise Kollagene [Prockop et al., Annu. Rev. Biochem. 64, 403 (1995), Wetzels et al., Am. J. Pathol. 139, 451 (1991)]; Ficolin [Ichijo et al., J. Biol. Chem. 268, 14505 (1993)]; Sialoprotein [Bellahcene et al., Cancer Res. 54, 2823 (1994)]; Laminin [von der Mark et al., Biochem. Biophys. Acta 823, 147 (1985); Hunt, Expl. Cell Biol. 57, 165 (1989)]; Proteoglykane [Schmidtchen et al., Biomed. Chromatography 7, 48 (1993)] oder Tenascin [Oyama et al., Cancer Res. 51, 4876 (1991); Herlyn et al., Cancer Res. 51, 4853 (1991)].

Der erfindungsgemäße Ligand (Teilstruktur B') kann beispielsweise ein Antikörper oder ein Antikörperfragment, wie beispielsweise der epitopbindende Teil eines Antikörpers, Fab, Fv, Einzelketten-Fv oder Fc sein, das spezifisch an ein Zellmembranantigen oder an ein Antigen auf der extrazellulären Matrix bindet, oder ein sonstiges Peptid oder Protein, welches an einen Rezeptor auf der jeweiligen Zellmembran bindet. Hierzu gehören beispielsweise Wachstumsfaktoren, Cytokine, Interferone, Tumornekrosefaktor Chemokine, deren Rezeptor-bindende Teilsequenzen, Peptidhormone, Angiotensin, Kinin oder Folsäure. Er kann auch ein Adhäsionsmolekül oder dessen Adhäsionssequenz sein, welche an ein korrespondierendes Molekül auf der Zellmembran oder auf der extrazellulären Matrix bindet oder der zielzellbindende Teil, ein extrazellulärer Teil eines Fc-Rezeptors, ein zielzellbindendes Glykoprotein eines Virus mit einem Tropismus für ausgewählte Zellen, oder eine an diese Zellen bindende Teilsequenz des Glykoproteins oder ein Peptid mit dessen Hilfe der Wirkstoff in der Zellmembran der exprimierenden Zelle verankert wird. Zu diesen verankernden Peptiden gehören beispielsweise Transmembrandomänen von Rezeptoren oder Virusproteinen oder Glykophospholipidanker.

Die Komponente d) kodiert für ein Peptid (Teilstruktur D), welches über die Teilstruktur C an das Protein B bzw. Protein B'B gebunden ist und so die Wirksamkeit des Proteins B inhibiert. Die Komponente d) kann eine beliebige Nukleinsäuresequenz darstellen. Bevorzugt werden jedoch Nukleinsäuresequenzen, welche für körpereigene Peptide oder Proteine kodieren, um die Gefahr einer Immunreaktion zu vermeiden oder zu vermindern. In einer weiteren bevorzugten Ausführungsform kodieren die im erfindungsgemäßen Strukturgen enthaltenen Komponenten b) und d) körpereigene Proteine bzw. Peptide.

In der Natur kommt eine beträchtliche Zahl von Proteinwirkstoffen in Form inaktiver Vorstufen (Protein BSD) vor. Eine solche Vorstufe wird dadurch aktiviert, daß Enzyme diese Vorstufe in eine Teilstruktur, welche den aktiven eigentlichen Proteinwirkstoff darstellt (Protein B) und in eine inaktive Teilstruktur (Teilstruktur D) spaltet. Die Spaltung dieser Vorstufe erfolgt an mindestens einer definierten Aminosäuresequenz, der sogenannten Spaltsequenz (Teilstruktur S).

Ein besonderer Gegenstand dieser Erfindung ist, daß diese natürlicherweise in Vorstufen von Proteinwirkstoffen vorkommende Spaltsequenz (Teilstruktur S) gegen die Teilstruktur C ausgetauscht ist. Dieser Austausch erfolgt dadurch, daß in der Nukleinsäuresequenz, welche für die naturgemäße Vorstufe (Protein BSD) kodiert, die Sequenz, kodierend für die Teilstruktur S, durch die Komponente c), kodierend für die Teilstruktur C, ersetzt wird. Nach Anfügung der Komponenten a) und ggf. b') entsteht ein erfindungsgemäßes Nukleinsäurekonstrukt bestehend aus z.B. den Komponenten a)b')b)c)d) bzw. a)b)c)d), dessen Expressionsprodukt Protein B'BCD bzw. BCD in der Teilstruktur C durch Proteasen, gebildet in Tumoren oder sezerniert von Tumorzellen oder Entzündungszellen, gespalten wird, so daß der aktive Wirkstoff Protein B'B oder B entstehen kann.

In einer weiteren Ausführungsform enthält das erfindungsgemäße Konstrukt mindestens zwei gleiche oder unterschiedliche Komponenten b)c)d) bzw. b')b)c)d), welche über eine sogenannte *"internal ribosomal entry site"* (IRES) miteinander verbunden sind.

Das erfindungsgemäße Nukleinsäurekonstrukt wird, eingefügt in einen nicht-viralen oder viralen Vektor, im allgemeinen zur Prophylaxe und/oder Therapie von Erkrankungen lokal verabreicht oder in den Kreislauf injiziert. Zu diesen Erkrankungen gehören insbesondere Tumorerkrankungen und Entzündungen. Derartige Entzündungen können beispielsweise durch physikochemische Schäden, durch eine Infektion oder durch eine Immunreaktion gegen körpereigenes oder fremdes Gewebe ausgelöst werden.

Ein weiterer Gegenstand der vorliegenden Erfindung bezieht sich daher auf die Verwendung eines erfindungsgemäßen Nukleinsäurekonstrukt zur Herstellung eines Heilmittels zur lokalen oder systemischen Verabreichung zur Prophylaxe und/oder Therapie von Tumoren, Leukämien, Allergien, Autoimmunerkrankungen, Infektionen, Entzündungen, Abstoßungsreaktionen von Transplantaten, Thrombosen, Gefäßverschlüssen, Störungen des Blutgerinnungs- und Blutkreislaufes, Verletzungen von Geweben und/oder Schäden des Nervensystems.

Die Auswahl der Komponenten des erfindungsgemäßen Nukleinsäurekonstruktes ist von der Erkrankung abhängig, welche durch die Verabreichung des Nukleinsäurekonstruktes therapiert werden soll, und kann wie folgt getroffen werden:

## Promotorsequenzen [Komponente a)]:

Gemäß der vorliegenden Erfindung sind zum einen Promotorsequenzen [Komponente a)] besonders bevorzugt, die uneingeschränkt aktivierbare Promotoren und Aktivatorsequenzen darstellen, wie beispielsweise der Promotor der RNA-Polymerase III, der Promotor der RNA-Polymerase II usw., der CMV-Promotor und -Enhancer oder der SV40 Promotor und zum anderen virale Promotor- und Aktivatorsequenzen, wie beispielsweise HBV, HCV, HSV, HPV, EBV, HTLV, HIV.

Beispielsweise kann beim HIV-Promotor die gesamte LTR-Sequenz einschließlich der TAR-Sequenz [Position ≤ -453 bis ≥ -80, Rosen et al., Cell 41, 813 (1985)] als virusspezifischer Promotor verwendet werden.

Ferner sind als Komponente a) metabolisch aktivierbare Promotor- und Enhancersequenzen, wie beispielsweise der durch Hypoxie induzierbare Enhancer, Zellzyklus-spezifisch aktivierbare Promotoren, wie beispielsweise der Promotor des cdc25C Gens, des Cyclin A Gens, des cdc2 Gens, des B-myb Gens, des DHFR-Gens oder des E2F-1 Gens oder Tetrazyklin-aktivierbare Promotoren, wie beispielsweise der Tetrazyklin-Operator in Kombination mit einem entsprechenden Repressor, besonders bevorzugt.

Gemäß der vorliegenden Erfindung sind als Promotorsequenzen auch Nukleotidsequenzen zu verwenden, welche nach Bindung von Transkriptionsfaktoren die Transkription eines am 3' Ende benachbart gelegenen Strukturgenes aktivieren.

Daneben sind als Komponente a) zellspezifisch-aktivierbare Promotoren besonders bevorzugt. Hierzu zählen bevorzugt Promotoren oder Aktivatorsequenzen aus Promotoren oder Enhancern von solchen Genen, die für Proteine bevorzugt in ausgewählten Zellen kodieren. Zum Beispiel sind in folgenden Zellen Promotoren für folgende Proteine bevorzugt zu verwenden:

Promotor- und Aktivatorsequenzen, die in Endothelzellen aktiviert werden, wie z.B. Hirn-spezifischer, endothelialer Glucose-1-Transporter, Endoglin, VEGF-Rezeptor-1 (flt-1), VEGF-Rezeptor-2 (flk-1, KDR), tiel-1 oder tiel-2, B61-Rezeptor (Eck-Rezeptor), B61, Endothelin, im speziellen, Endothelin B, Endothelin-1, Endothelin-Rezeptoren, insbesondere der Endothelin B-Rezeptor, Mannose-6-Phosphat-Rezeptoren, von Willebrand Faktor, Il-1α, Il-1β, Il-1-Rezeptor, Vascular Cell Adhesion Molecule (VCAM-1) oder synthetische Aktivatorsequenzen.

Als Alternative zu natürlichen endothelzellspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist [Lee et al., Biol. Chem. 266, 16188 (1991), Dormann et al., J. Biol. Chem. 267, 1279 (1992) und Wilson et al., Mol. Cell Biol. 10, 4854 (1990)].

Promotoren oder Aktivatorsequenzen, die in Zellen in Nachbarschaft aktivierter Endothelzellen aktiviert werden, insbesondere in glatten Muskelzellen, sind z.B. im VEGF-Gen enthalten. Die genregulatorischen Sequenzen für das VEGF-Gen sind die 5' flankierende Region, die 3' flankierende Region, das c-Src-Gen oder das v-Src-Gen.

Darüber hinaus eignen sich Steroid-Hormonrezeptoren und deren Promotorelemente, insbesondere der Maus-Mammatumor-Virus-Promotor, oder Promotorelemente des Gens kodierend für Tropomyosin, α-Actin, α-Myosin, Rezeptor für PDGF, Rezeptor für FGF, MRF-4, Phosphofructokinase A, Phosphoglyceratemutase, Troponin C, Myogenen, Rezeptoren für Endothelin A, Desmin oder separate "artifizielle" Promotoren. Daneben eignen sich Promotorelemente, an die Faktoren der Helix-Loop-Helix (HLH)-Familie (MyoD, Myf-5, Myogenen, MRF4 [Übersicht in Olson and Klein, Genes Dev. 8, 1 (1994)] als muskelspezifische Transkriptionsfaktoren binden können. Des weiteren gehören zu den muskelspezifischen Transkriptionsfaktoren das Zinkfingerprotein GATA-4 [Arceci et al., Mol. Cell Biol. 13, 2235 (1993), Ip et al., Mol. Cell Biol. 14, 7517 (1994)] sowie die Gruppen der MEF-Transkriptionsfaktoren [Yu et al., Gene Dev. 6, 1783 (1992)].

Die HLH-Proteine sowie GATA-4 zeigen ebenso eine muskelspezifische Transkription nicht nur mit Promotoren von muskelspezifischen Genen, sondern auch im heterologen Kontext, so auch mit "artifiziellen" Promotoren. Derartige artifizielle Promotoren sind beispielsweise multiple Kopien der (DNA) Bindestelle für muskelspezifische HLH-Proteine wie der E-Box (Myo D), z.19. 4x AGCAGGTGTTGGGAGGC, [Weintraub et al., PNAS 87, 5623 (1990)] oder multiple Kopien der DNA Bindestelle für GATA-4 des α-Myosin-Heavy Chain Gens, z.B. 5'-GGCCGATGGGCAGATAGAGGGGGCCGATGGGCAGATAGAGG3' [Molkentin et al., Mol. Cell Biol. 14, 4947 (1994)].

Promotoren und Aktivatorsequenzen, die in Leukämiezellen aktiviert werden, sind beispielsweise Promotoren für c-myc, HSP-70, bcl-1/cyclin D-1, bcl-2, IL-6, IL-10, TNFα, TNFβ, HOX-11, BCR-Abl, E2A-PBX-1 oder PML-RATA.

Promotoren oder Aktivatorsequenzen, die in Tumorzellen aktiviert werden, ist beispielsweise eine Promotor- oder Aktivatorsequenz, mit der Transkriptionsfaktoren interagieren, die in Tumorzellen gebildet werden oder aktiv sind. Hierzu zählen zu den bevorzugten Promotoren oder Aktivatorsequenzen genregulatorische Sequenzen bzw. Elemente aus Genen, die besonders in Krebszellen oder Sarkomzellen gebildete Proteine kodieren. So wird bei kleinzelligen Bronchialkarzinomen beispielsweise der Promotor des N-CAM-Proteins, bei Ovarialkarzinomen der Promotor des "Hepatitis growth factor"-Rezeptors oder des L-Plastin und bei Pankreaskarzinomen der Promotor des L-Plastins oder des polymorphen epithelialen Mucins (PEM) verwendet.

Promotoren und Aktivatorsequenzen, die in Gliazellen aktiviert werden, sind insbesonders die genregulatorischen Sequenzen bzw. Elemente aus Genen, die beispielsweise für folgende Proteine kodieren: das Schwannzell-spezifische Protein Periaxin, Glutaminsynthetase, das Gliazell-spezifische Protein (Glial fibrillary acid protein = GFAP), das Gliazellprotein S100b, IL-6 (CNTF), 5-HT-Rezeptoren, TNFα, IL-10, Insulin-like Growth Factor Receptor I and II oder VEGF. Die genregulatorischen Sequenzen für das VEGF-Gen sind bereits oben aufgeführt worden.

Promotoren und Aktivatorsequenzen, die in Lymphozyten und/oder Makrophagen aktiviert werden, sind beispielsweise die Promotor- und Aktivatorsequenzen der Gene kodierend für Cytokine, Cytokinrezeptoren und Adhäsionsmoleküle und Rezeptoren für das Fc-Fragment von Antikörpern. Hierzu gehören beispielsweise: IL-1-Rezeptor, IL-1α, IL-1β, IL-2, IL-2-Rezeptor, IL-3, IL-3-Rezeptor (α-subunit), IL-3-Rezeptor (β-subunit), IL-4, IL-4-Rezeptor, IL-5, IL-6, IL-6-Rezeptor, Interferon Regulatory Factor 1 (IRF-1), (der Promotor von IRF-1 wird durch IL-6 gleichermaßen aktiviert wie durch IFNγ oder IFNβ), IFNγ Responsive Promotor, IL-7, IL-8, IL-10, IL-11, IFNγ, GM-CSF, GM-CSF-Rezeptor (α-Kette), IL-13, LIF, Makrophagen-Colony Stimulating Factor (M-CSF)-Rezeptor, Typ I und II Makrophagen Scavenger Rezeptoren, MAC-1 (Leukozytenfunktionsantigen), L F A - 1 α (Leukozytenfunktionsantigen) oder p150,95 (Leukozytenfunktionsantigen).

Promotor- und Aktivatorsequenzen, die in Synovialzellen aktiviert werden, sind beispielsweise die Promotorsequenzen für Matrix-Metalloproteinasen (MMP), wie z.B. für: MMP-1 (interstitielle Kollagenase), oder MMP-3 (Stromelysin/Transin). Hierzu gehören des weiteren die Promotorsequenzen für "Tissue Inhibitors of Metalloproteinases" (TIMP), beispielsweise TIMP-1 TIMP-2, TIMP-3.

Gemäß der vorliegenden Erfindung können mehrere der beispielhaft aufgeführten Promotorsequenzen miteinander kombiniert werden, um eine möglichst hohe Zielzellspezifität in der Expression des erfindungsgemäßen Nukleinsäurekonstruktes zu erreichen. Es kann auch die Kombination zweier gleicher Promotoren erfolgen. Eine Kombination von mehreren Promotorsequenzen kann beispielsweise erfolgen über chimäre Promotoren oder hybride Promotoren. Ein chimärer Promotor stellt die Kombination einer stromaufwärts gelegenen zellspezifisch, metabolisch oder virusspezifisch aktivierbaren Aktivatorsequenz mit einem stromabwärts gelegenen Promotormodul dar, welches die Transkriptionsfaktoren der Familien CDF und CHF oder E2F und CHF bindet und hierdurch die Aktivierung der stromaufwärts gelegenen Aktivatorsequenz in G0- und G1-Phase des Zellzyklus hemmen kann [Lucibello et al., EMBO J. 14, 132 (1994)].

Bei hybride Promotoren ist beispielsweise die TATA-Box eines Promotors mutiert, wobei diese Mutation durch eine korrespondierende Mutation im Gen eines TATA-Bindeproteins kompensiert wird und dieses TATA-Bindeprotein unter der Kontrolle eines weiteren Promotors steht.

## Nukleinsäuresequenz [Komponente b')], die für einen Liganden (Teilstruktur B') kodiert:

Gemäß der vorliegenden Erfindung ist der Ligand eine Substanz, welche ein Membranantigen an einen Rezeptor oder an ein Adhäsionsmolekül auf der Zielzelle bindet oder die in der Zellmembran integriert ist und/oder an die extrazelluläre Matrix bindet. Eine Übersicht über die wesentlichen Cytokine und Wachstumsfaktoren und deren Rezeptoren, Adhäsionsmoleküle und extrazelluläre Matrix-Proteine gibt von Ayad et al., The Extracellular Matrix, Academic Press 1994; Callard et al., The Cytokine, Academic Press 1994; Pigott et al., The Adhesion Molecule, Academic Press 1994, und Barclay et al., The Leucocyte Antigen, Academic Press 1994.

Beispielsweise sind an Rezeptoren bindende Substanzen Wachstumsfaktoren wie VEGF, PDGF, EGF, TGFα, TGFβ, KGF, SDGF, FGF, IGF, HGF, NGF, BDNF, Neurotrophine, BMF, Bombesin, M-CSF, Thrombopoietin, Erythropoietin, SCF, SDGF, Oncostatin, PDEGF, Endothelin-1, Cytokine wie IL-1, IL-2, IL-3, IL-4, IL-5, IL-6, IL-7, IL-8, IL-9, IL-10, IL-11, IL-12, IL-13, IL-14, IL-15, Interferon α, β und γ, Tumornekrosisfaktoren TNFα, - β, Chemokine wie RANTES, MCAF, MIP-1α oder -β, NAP, β-Thromboglobulin, Peptidhormone wie SRH, SIH oder STH, MRH oder MSH, PRH, PIH oder Prolaktin, LH-RH, FSH-RH, LH/ICSH oder FSH, TRH oder TSH, CRH oder ACTH, Angiotensin, Kinine, Homologe oder Analoge hiervon oder Vitamine wie z.B. Folsäure.

Gemäß der vorliegenden Erfindung kann der Ligand auch ein Adhäsionsmolekül, ein Teil eines Adhäsionsmoleküls oder ein Analogon eines Adhäsionsmoleküls sein, welches an ein korrespondierendes zellmembranständiges Adhäsionsmolekül oder an eine andere spezifische Bindestruktur für ein Adhäsionsmolekül auf der Zielzelle oder der extrazellulären Matrix bindet.

Derartige als Liganden funktionsfähige Adhäsionsmoleküle sind beispielsweise Lewis X (für GMP-140), S-Lewis X (für ELAM-1), LFA-1 (für ICAM-1 und ICAM-2), MAC-1 (für ICAM-1), VLA-4 (für VCAM-1), PECAM (für PECAM), Vitronectin (für den Vitronectinrezeptor), GMP-140 (für Lewis X), ICAM-1, ICAM-2 (für LFA-1, MAC-1), VCAM-1 (für VLA-4), Fibronectin (für VLA-4), Laminin (für VLA-6), Laminin (für VLA-1, VLA-2, VLA-3), Fibrinogen (für GPIIb-IIIa), B7 (für CD28), CD28 (für B7), CD40 (für CD40L) oder CD40L (für CD40).

Gemäß der vorliegenden Erfindung kann der Ligand auch der extrazelluläre Teil eines Fc-Rezeptors sein [Dougherty et al., Transfusion Science 17, 121 (1996)]. Des weiteren kann der Ligand auch ein Antikörpermolekül oder der epitopbindende Teil eines Antikörpermoleküls sein. Die murinen monoklonalen Antikörper sind bevorzugt in humanisierter Form einzusetzen. Die Humanisierung erfolgt in der von Winter et al. [Nature 349, 293 (1991)] und Hoogenbooms et al. [(Rev. Tr. Transfus. Hemobiol. 36, 19 1993)] dargestellten Weise.

Rekombinante Antikörperfragmente werden direkt aus existierenden Hybridomen hergestellt oder werden mit Hilfe der "phage display"-Technologie [Smith, Science 228, 1315 (1985)] aus Bibliotheken muriner bzw. humaner Antikörperfragmente isoliert [Winter et al., Annu. Rev. Immunol. 12, 433 (1994)]. Diese Antikörperfragmente werden dann auf genetischer Ebene direkt für weitere Manipulationen, z.B. für die Fusion mit anderen Proteinen eingesetzt.

Zur Herstellung von rekombinanten Antikörperfragmenten aus Hybridomen wird die genetische Information, die für die antigenbindenden Domänen (VH, VL) der Antikörper kodiert, durch Isolierung der mRNA, die reverse Transkription der RNA in cDNA und die anschließende Amplifikation mittels Polymerasekettenreaktion [Saiki et al., Science 230, 1350 (1985)] und Oligonukleotiden komplementär zu den 5'- bzw. 3'-Enden der variablen Fragmente (Orlandi et al., 1989) gewonnen. Die VH- und VL-Fragmente werden dann in bakterielle Expressionsvektoren z.B. in Form von Fv-Fragmenten [Skerra & Plückthun, Science 240, 1038 (1988)], einzelkettigen Fv-Fragmenten (scFv) [Bird et al., Science 242, 423 (1988), Huston et al., PNAS-USA 85, 5879 (1988)] oder als Fab-Fragmente [Better et al., Science 240, 1041 (1988)] kloniert.

Neue Antikörperfragmente können mittels der "phage-display"-Technologie auch direkt aus Antikörperbibliotheken (Immunbibliotheken, naive Bibliotheken) murinen oder humanen Ursprungs isoliert werden. Beim "phage display" von Antikörperfragmenten werden die antigenbindenden Domänen als Fusionsproteine mit dem Hüllprotein g3P filamentöser Bakteriophagen entweder in das Phagengenom [McCafferty et al., Nature 348, 552 (1990)] oder in Phagemid-Vektoren [Breitling et al., Gene 104, 147 (1991)] in Form von scFv-Fragmenten [MacCafferty et al. Nature 348, 552 (1990)] oder als Fab-Fragmente [Hoogenboom et al., Nucl. Acid Res. 19, 4133 (1991), Barbas et al., PNAS-USA 88, 7978 (1991)] kloniert. Antigen-bindende Phagen werden an antigenbeladenen Plastikgefäßen (panning) [Marks et al., J. Mol. Biol. 222, 581 (1991)], an antigenkonjugierten, paramagnetischen "beads" [Hawkins et al., J. Mol. Biol. 226, 889 (1992)] oder durch Bindung an Zelloberflächen [Marks et al., Bio/Technol. 11, 1145 (1993)] selektioniert.

Immunbibliotheken werden durch PCR-Amplifikation der variablen Antikörperfragmente aus B-Lymphozyten immunisierter Tiere [Sastry et al., PNAS-USA 86, 5728 (1989), Ward et al., Nature 341, 544 (1989), Clackson et al., Nature 352, 624 (1991)] oder Patienten hergestellt [Mullinax et al., PNAS-USA 87, 8095 (1990), Barbas et al., PNAS-USA 88, 7978 (1991)]. Dazu werden Kombinationen von Oligonukleotiden, die spezifisch für murine [Orlandi et al., PNAS-USA 86, 3833 (1989), Sastry et al., PNAS-USA 86, 5728 (1989)] oder humane Immunglobulingene [Larrick et al., BBRC 160, 1250 (1989)] bzw. für die humanen Immunglobulin-Genfamilien [Marks et al. Eur. J. Immunol. 21, 985 (1991)] sind, verwendet.

Naive Bibliotheken lassen sich beispielsweise von nichtimmunisierten Spendern als Quelle der Immunglobulingene herstellen [Marks et al., J. Mol. Biol. 222, 581 (1991)]. Alternativ können Immunglobulin-Keimbahngene zur Herstellung semisynthetischer Antikörperrepertoires eingesetzt werden, wobei die Komplementarität-bestimmende Region 3 der variablen Fragmente durch PCR mit Hilfe degenerierter Primer ergänzt wird [Hoogenboom & Winter, J. Mol. Biol. 227, 381 (1992), Barbas et al., PNAS-USA 89, 4457 (1992), Nissim et al., EMBO J. 13, 692 (1994), Griffiths et al., EMBO J. 13, 3245 (1994)]. Diese sogenannten "single pot"-Bibliotheken haben gegenüber Immunbibliotheken den Vorteil, daß Antikörperfragmente gegen eine Vielzahl von Antigenen aus einer einzigen Bibliothek isoliert werden können [Nissim et al., EMBO J. 13, 692 (1994)].

Die Affinität von Antikörperfragmenten kann mittels der "phage display"-Technologie weiter erhöht werden, wobei neue Bibliotheken von bereits existierenden Antikörperfragmenten durch zufällige [Hawkins et al., J. Mol. Biol. 226, 889 (1992), Gram et al., PNAS-USA 89, 3576 (1992)], kodonbasierende [Glaser et al., J. Immunol. 149, 3903 (1992)] oder gezielte Mutagenese [Balint & Larrick, Gene 137, 109 (1993)], durch "chain shuffling" einzelner Domänen mit Fragmenten aus naiven Repertoires [Marks et al., Bio/Technol. 10, 779 (1992)] oder unter Zuhilfenahme von bakteriellen Mutatorstämmen [Low et al., J. Mol. Biol. 260, 359 (1996)] hergestellt werden und durch Reselektion unter stringenten Bedingungen [Hawkins et al., J. Mol. Biol. 226, 889 (1992)] Antikörperfragmente mit verbesserten Eigenschaften isoliert werden. Zusätzlich können murine Antikörperfragmente durch einen stufenweisen Austausch einer der variablen Domänen gegen ein humanes Repertoire und anschließende Selektion mit dem ursprünglichen Antigen ("guided selection") [Jespers et al., Bio/Technol. 12, 889, 1994)] humanisiert werden. Alternativ erfolgt die Humanisierung muriner Antikörper durch zielgerichteten Austausch der hypervariablen Regionen humaner Antikörper durch die korrespondierenden Regionen des originalen murinen Antikörpers [Jones et al., Nature 321, 522 (1987)].

Gemäß der vorliegenden Erfindung kann der Ligand auch die Nukleotidsequenz kodierend für ein Hüllprotein oder einen Teil des Hüllproteins von Viren darstellen, welche über ihr Hüllprotein an ausgewählte Zellen spezifisch binden.

Des weiteren kann der Ligand ein Peptid sein, mit dessen Hilfe der Wirkstoff (Protein B) in der Zellmembran der exprimierenden Zellen verankert wird. Zu diesen verankernden Peptiden gehören Transmembrandomänen von zellmembranständigen Rezeptoren oder von Virusproteinen, wie beispielsweise die Transmembransequenz des menschlichen Makrophagengenkolonie-stimulierenden Faktors [DNA-Position $\leq$ 1485 bis $\geq$ 1554; Cosman et al., Behring Inst. Mitt. 83, 15 (1988)] oder die DNA-Sequenz für die Signal- und Transmembranregion des menschlichen Respiratory Syncytial Virus (RSV)-Glykoproteins G [Aminosäuren 1 bis 63 oder deren Teilsequenzen, Aminosäuren 38 bis 63; Vijaya et al., Mol. Cell Biol. 8, 1709 (1988); Lichtenstein et al., J. Gen. Virol. 77, 109 (1996)] oder die DNA-Sequenz für die Signal- und Transmembranregion der Influenzavirus-Neuraminidase [Aminosäuren 7 bis 35 oder die Teilsequenz Aminosäuren 7 bis 27, Brown et al., J. Virol 62, 3824 (1988)].

Zur Verankerung des Wirkstoffes in die Zellmembran der den Wirkstoff bildenden transduzierten Zellen kann jedoch auch die Nukleotidsequenz für einen Glykophospholipid-Anker [Übersicht über Glykophospholipid verankerte Membranproteine bei Ferguson et al., Ann. Rev. Biochem. 57, 285 (1988)] eingefügt werden. Glykophospholipid-Anker sind beispielsweise für das CEA [DNA-Position < 893 bis > 1079; Berling et al., Cancer Res. 50, 6534 (1990)], für das N-CAM [Cunningham et al., Science 236, 799 (1987)] und für weitere Membranproteine wie beispielsweise Thy-1 [Clissold, Biochem. J. 281, 129 (1992)] oder CD16 [Selvaray et al., Nature 333, 565 (1988)] beschrieben worden.

Die Wahl des Liganden richtet sich in erster Linie nach der Zielzelle, welche durch das Nukleinsäurekonstrukt transduziert werden soll. Als Beispiele hierfür gelten Liganden für aktivierte Endothelzellen. Hierzu gehören im Sinne der Erfindung Antikörper oder Antikörperfragmente, die gegen Membranstrukturen von Endothelzellen gerichtet sind, wie sie beispielsweise von Burrows et al., Pharmac. Ther. 64, 155 (1994), Hughes et al. Cancer Res. 49, 6214 (1989) und Maruyama et al. PNAS-USA 87, 5744 (1990) beschrieben wurden. Insbesondere zählen hierzu Antikörper gegen Actin, Angiotensin II-Rezeptoren, Antikörper gegen Rezeptoren für Wachstumsfaktoren wie VEGF, FGF, PDGF oder EGF und Antikörper gegen Adhäsionsmoleküle wie beispielsweise gegen den Vitronectinrezeptor oder ICAM-3.

Zu den Liganden gehören des weiteren alle Wirkstoffe, welche an Membranstrukturen oder Membranrezeptoren auf Endothelzellen binden. Beispielsweise gehören hierzu IL-1 oder Wachstumsfaktoren oder deren Fragmente bzw. Teilsequenzen von ihnen, die an Rezeptoren, welche in Endothelzellen exprimiert werden, binden, wie beispielsweise PDGF, bFGF, VEGF oder TGFβ [Pusztain et al., J. Pathol. 169, 191 (1993)].

Des weiteren gehören hierzu Adhäsionsmoleküle, welche an aktivierte und/oder proliferierende Endothelzellen binden. Derartige Adhäsionsmoleküle, wie beispielsweise Slex, LFA-1, MAC-1, LECAM-1, VLA-4 oder Vitronectin wurden bereits beschrieben [Augustin-Voss et al., J. Cell Biol. 119, 483 (1992), Pauli et al., Cancer Metast. Rev. 9, 175 (1990), Honn et al., Cancer Metast. Rev. 11, 353 (1992), Pigott et al., The Adhesion Molecule, Academic Press (1994)].

Zu den Liganden im Sinne dieser Erfindung gehören insbesonders auch Glykoproteine der Hülle von Viren, die einen Tropismus für Endothelzellen haben. Zu diesen Viren gehören beispielsweise Filoviren, wie z.B. das Marburg-Virus mit seinem Hüllprotein GP (glycoprotein) und sGP (second glycoprotein) oder das Ebola-Virus jeweils mit seinem Hüllprotein GP und sG, das Cytomegalovirus besonders mit seinem gB-Protein, das Herpes Simplex-Virus Type I, das HIV-1 Virus, das Masern-Virus, das Hantaan-Virus, Alphaviren, wie Semliki Forest-Virus, das Virus des epidemischen, haemorrhagischen Fiebers, das Poliovirus oder Enteroviren, wie z.B. ECHO 9, ECHO 12, Coxsackie B3.

Als Beispiele für Liganden für Muskelzellen sind Antikörper oder Antikörperfragmente zu nennen, die gegen Membranstrukturen von Muskelzellen, insbesondere von glatten Muskelzellen gerichtet sind. Derartige Antikörper sind beispielsweise der Antikörper 10F3, Antikörper gegen Actin, Antikörper gegen Angiotensin II-Rezeptoren, Antikörper gegen Rezeptoren für Wachstumsfaktoren oder Antikörper beispielsweise gegen EGF-Rezeptoren, gegen PDGF-Rezeptoren, gegen FGF-Rezeptoren oder Antikörper gegen Endothelin A-Rezeptoren.

Zu den Liganden gehören des weiteren Nukleotidsequenzen für Wirksubstanzen, welche an Membranstrukturen oder Membranrezeptoren auf Muskelzellen binden [Pusztai et al., J. Pathol. 169, 191 (1993), Harris, Curr. Opin. Biotechnol. 2, 260 (1991)]. Beispielsweise gehören hierzu Wachstumsfaktoren oder deren Fragmente bzw. Teilsequenzen von ihnen, die an Rezeptoren, welche in glatte Muskelzellen exprimiert werden, binden, wie beispielsweise PDGF, EGF,TGFβ, TGFα, FGF oder Endothelin A.

Zu den Liganden gehören auch Glykoproteine der Hülle von solchen Viren, die einen Tropismus für Muskelzellen haben. Zu diesen Viren gehört beispielsweise das Cytomegalovirus [Speir et al., Science 265, 391 (1994)].

Als Beispiel für Liganden für aktivierte Makrophagen und/oder aktivierte Lymphozyten sind des weiteren Nukleotidsequenzen, die für Substanzen kodieren, welche an die Oberfläche von Immunzellen spezifisch binden. Hierzu gehören Antikörper oder Antikörperfragmente, die gegen Membranstrukturen von Immunzellen gerichtet sind, wie sie beispielsweise von Powelson et al., Biotech. Adv. 11, 725 (1993) und Barclay et al., The Leucocyte Antigen, Academic Press (1994) beschrieben wurden. Des weiteren gehören zu den Liganden auch monoklonale oder polyklonale Antikörper oder Antikörperfragmente, die mit ihrem antigenbindenden variablen Teil an Fc-$\gamma$ - oder Fc-$\varepsilon$ - oder Fc-$\mu$ Rezeptoren von Immunzellen binden [Rojanasakul et al., Pharm. Res. 11, 1731 (1994)]. Des weiteren gehört hierzu das Fc-Fragment von humanem monoklonalem oder polyklonalem Immunglobulin.

Zu den Liganden gehören des weiteren alle Substanzen, welche an Membranrezeptoren auf der Oberfläche von Immunzellen binden. Hierzu gehören Zytokine wie beispielsweise IL-1, IL-2, IL-3, IL-4, IL-6, IL-10, TNF$\alpha$, GM-CSF, M-CSF, des weiteren Wachstumsfaktoren wie beispielsweise EGF, TGF, FGF, IGF oder PDGF oder deren Fragmente bzw. Teilsequenzen von ihnen, die an Rezeptoren, welche in Immunzellen exprimiert werden, binden [Callard et al., The Cytokine, Academic Press (1994)]. Hierzu gehören des weiteren Adhäsionsmoleküle und andere Liganden, welche an Zellmembranstrukturen, auf Makrophagen, in Milz, Leber, Lunge und andere Gewebe binden [Pigott et al., The Adhesion Molecule, Academic Press (1994), Perales et al., Eur. J. Biochem. 226, 255 (1994)].

Zu den Liganden im Sinne dieser Erfindung gehören auch Glykoproteine der Hülle von solchen Viren, die einen Tropismus für Lymphozyten und/oder Makrophagen haben. Zu diesen Makrophagen infizierenden Viren gehören beispielsweise HIV-1, besonders solche Stämme mit Mutationen in der V3-Region von gp120, die zu einer erhöhten Bindung an Makrophagen führen, HIV-2, Hantaviren, beispielsweise des Punmalavirus, Cytomegalovirus, Respiratory Syncytial Virus, Herpes simplex-Virus oder Filoviren.

Zu den Lymphozyten infizierenden Viren gehören beispielsweise Varizella-Zoster-Virus (VZV), da VZV besonders T-Zellen infiziert, Herpes Virus 6 (HHV-6), da HHV-6 ebenso besonders T-Zellen infiziert, Rabies-Virus, da das Rabies-Virus Hüllprotein besonders an TH2-Zellen bindet, HIV-1, da das Glykoprotein gp120 bevorzugt an das CD4 Molekül von T-Zellen bindet, HTLV-II, da HTLV-II besonders B-Zellen infiziert, HTLV-I, da HTLV-I besonders T-Zellen infiziert, Influenza C-Viren, da Influenza-C-Viren über das Haemagglutinin-Esterase-Fusions-(HEF)-Protein an N-acetyl-9-$\beta$-acetylneuraminsäure (Neu 5,9 Ac) binden, welche bevorzugt auf B-Lymphzyten, weniger oder nicht auf T-Lymphozyten vorkommt, Influenza C-Viren mit Mutation in der Nukleotidposition 872, die die Position 284 des HEF der Aminosäuresequenz kodiert, beispielsweise mit einem Austausch des Threonins durch Isoleucin, da das Oberflächenprotein HEF mit dieser Mutation hat eine deutlich stärkere Affinität zum N-acetyl-9-0-acetylneuraminsäure-Rezeptor als das Wildvirus hat, HEF Spaltprodukte des Influenza C-Virus, welche die Bindestruktur für N-acetyl-9-$\beta$-acetylneuraminsäure enthalten. Diese Bindestruktur ist durch die katalytische Triade Serin-71, Histidin 368 oder - 369 und Asparaginsäure 261 definiert, Epstein-Barr Virus, da EBV besonders B-Zellen infiziert, Herpes simplex-Virus-2, da HSV-2 infiziert besonders T-Zellen infiziert oder Masernvirus.

Als Beispiele für Liganden für Synovialzellen und Entzündungszellen sind Nukleinsäuresequenzen zu nennen, die für Antikörper oder Antikörperfragmente kodieren, die mit ihren variablen Domänen an Membranstrukturen von Synovialzellen oder Entzündungszellen binden. Solche Membranstrukturen sind beispielsweise Vimentin [Miettinen et al., Am. J. Pathol. 117, 18 (1984)], Fibronectin [Wojciak et al., Clin. Exp. Immunol. 93, 108 (1993)] oder Fc-Rezeptoren. Hierzu gehören auch Antikörper oder Antikörperfragmente, die mit ihren konstanten Domänen an Fc-Rezeptor binden [Rojanasakul et al., Pharm. Res. 11, 1731 (1994)].

Hierzu gehören des weiteren alle Wirkstoffe, welche an Membranstrukturen oder Membranrezeptoren auf Synovialzellen binden. Beispielsweise gehören hier Cytokine oder Wachstumsfaktoren oder deren Fragmente bzw. Teilsequenzen von ihnen, die an Rezeptoren, welche durch Synovialzellen exprimiert werden, binden, wie beispielsweise IL-1-RA, TNF$\alpha$, IL-4, IL-6, IL-10, IGF, TGF$\beta$ [Callard et al., The Cytokine, Academic Press (1994)].

Als Beispiele für Liganden für mit Viren infizierte Zellen sind Nukleinsäurekonstrukte zu nennen, die für Antikörper oder Antikörperfragmente kodieren, die gegen die Virusantigene gerichtet sind, die auf der Zellmembran von virusinfizierten Zellen lokalisiert sind. Derartige Antikörper sind beispielsweise gegen Antigene von HBV, HCV, HSV, HPV, HIV, EBV oder HTLV gerichtet.

Als Beispiel für Liganden für Leberzellen und weitere Gewebezellen gehören alle Substanzen, welche an Membranstrukturen oder Membranrezeptoren auf der Oberfläche von Leberzellen binden. Beispielsweise gehören hierzu Wachstumsfaktoren, wie Zytokine, EGF, TGF, FGF oder PDGF, oder deren Fragmente bzw. Teilsequenzen von ihnen, die an Rezeptoren, welche in derartigen Zellen exprimiert werden, binden.

Hierzu gehören des weiteren Liganden, welche an Zellmembranstrukturen binden, welche für bestimmte Gewebe selektiv sind. Hierzu zählen beispielsweise:

| Membranstruktur | Ligand | Gewebezellen |
|---|---|---|
| Transferrin-Rezeptor | Transferrin | Leber, andere Gewebezellen |
| Insulin-Rezeptor | Insulin | Leber, andere Gewebezellen |
| Fc-γ-Rezeptoren | Immunglobulin G | retikuloendotheliales System, andere Gewebezellen |

Diese Liganden und Membranstrukturen sind übersichtlich bei Perales et al., Eur. J. Biochem. 226, 255 (1994) beschrieben.

Zu den Liganden gehören besonders Glykoproteine der Hülle von Viren, die einen Tropismus für ausgewählte Zellen haben, wie beispielsweise für Bronchialepithelzellen (Respiratory syncytial virus), Leberzellen (Hepatitis C-Virus), Filoviren, Marburg-Virus über den Asialoglykoprotein-Rezeptor von Leberzellen, Hepatitis B-Virus, wobei Leberzellen bevorzugt über den Asialoglykoprotein-Rezeptor an der preS2 und preS1 Domäne von HBV binden, Hepatitis D-Virus, lebersinusoidale Zellen, Hepatitis B-Virus wobei HBV über Fibronectin gebunden wird.

Als Beispiele für Liganden für Gliazellen gehören Nukleinsäuresequenzen, die für Antikörper oder Antikörperfragmente kodieren, die gegen Membranstrukturen von Gliazellen gerichtet sind, wie sie beispielsweise von Mirsky et al. [Cell and Tissue Res. 240, 723 (1985)], Coakham et al. [Prog. Exp. Tumor Res. 29, 57 (1985)] und McKeever et al. [Neurobiol. 6, 119 (1991)] berichtet wurden. Zu diesen Membranstrukturen gehören des weiteren Neuraladhäsionsmoleküle wie N-CAM, insbesondere dessen Polypeptidkette C [Nybroe et al., J. Cell Biol. 101, 2310 (1985)]. Hierzu gehören des weiteren alle Wirkstoffe, welche an Membranstrukturen oder Membranrezeptoren auf Gliazellen binden. Beispielsweise gehören hierzu Insulin und Insulin-like growth factor und diejenigen Fragmente dieser Wachstumsfaktoren, welche an die zugehörigen Membranrezeptoren binden.

Zu den Liganden im Sinne der Erfindung gehören des weiteren Nukleinsäuresequenzen die für Glykoproteine der Hülle von solchen Viren kodieren, die einen Tropismus für Gliazellen haben.

Zu diesen Viren gehören beispielsweise HIV-1 Subtyp JRF1 oder Herpes simplex-Virus I.

Als Beispiele für Liganden für Leukämiezellen gehören Nukleinsäurekonstrukte, die für Antikörper oder Antikörperfragmente kodieren, die gegen Membranstrukturen von Leukämiezellen gerichtet sind. Eine große Anzahl derartiger monoklonaler Antikörper sind bereits für diagnostische und therapeutische Verfahren beschrieben worden [Kristensen, Danish Medical Bulletin 41, 52 (1994); Schranz, Therapia Hungarica 38, 3 (1990); Drexler et al., Leuk. Res. 10, 279 (1986); Naeim, Dis. Markers 7, 1 (1989); Stickney et al., Curr. Opin. Oncol. 4, 847 (1992); Drexler et al., Blut 57, 327 (1988); Freedman e al., Cancer Invest. 9, 69 (1991)]. Je nach Typ der Leukämie sind die Liganden beispielsweise monoklonale Antikörper oder deren antigenbindende Antikörperfragmente folgender Spezifität geeignet:

AML-Zellen mit den Membranantigenen CD13, CD14, CD15, CD33, CA-MAL sowie Sialosyl-Le; B-CLL-Zellen mit den Membranantigenen CD5, CD1c, CD23 sowie Idiotypen und Isotypen der Membranimmunglobuline; T-CLL-Zellen mit den Membranantigenen CD33, M38, IL-2-Rezeptoren sowie T-Zell-Rezeptoren; und ALL-Zellen mit den Membranantigenen CALLA, CD19 sowie Non-Hodgkin Lymphoma.

Zu den Liganden gehören des weiteren alle Wirkstoffe, welche an Membranstrukturen oder Membranrezeptoren von Leukämiezellen binden. Beispielsweise gehören hierzu Wachstumsfaktoren oder deren Fragmente bzw. Teilsequenzen von ihnen, die an Rezeptoren, welche in Leukämiezellen exprimiert werden, binden.

Derartige Wachstumsfaktoren wurden bereits beschrieben [Übersichten bei Cross et al., Cell 64, 271 (1991); Aulitzky et al., Drugs 48, 667 (1994); Moore, Clin. Cancer Res. 1, 3 (1995); Van Kooten et al., Leuk. Lymph. 12, 27 (1993)]. Beispielsweise gehören zu ihnen IFNα bei Non-Hodgkin Lymphomen, IL-2, besonders bei T-Zell-Leukämien, FGF bei T-Zell-, monozytären, myeloiden, erythroiden und megakaryoblastischen Leukämien, TGFβ bei Leukämien oder Retinoide, z.B. "Retinoic acid" bei akuter promyelozytärer Leukämie.

Als Beispiele für Liganden für Tumorzellen gehören Nukleinsäuresequenzen, die für Antikörper und Fragmente dieser Antikörper kodieren, welche gegen Membranstrukturen auf Tumorzellen gerichtet sind. Derartige Antikörper wurden zum Beispiel von Sedlacek et al., Contrib. to Oncol. 32, Karger Verlag, München (1988) und Contrib. to Oncol. 43, Karger Verlag, München (1992) übersichtlich dargestellt.

Weitere Beispiele stellen Antikörper gegen Sialyl Lewis, Peptide auf Tumoren, welche von T-Zellen erkannt werden, von Onkogenen exprimierte Proteine, Ganglioside wie GD3, GD2, GM2, 9-O-acetyl-GD3, Fucosyl-GM1, Blutgruppenantigene und deren Vorläufer, Antigene auf dem polymorphen epithelialen Mucin oder Antigene auf Heat Shock Proteinen dar.

## Nukleinsäuresequenz [Komponente b)], die für einen Wirkstoff (Protein B) kodiert:

Der Wirkstoff (Protein B) gemäß der vorliegenden Erfindung kann eine Substanz sein, welche beispielsweise in eine biologische Aktivierungskaskade eingreift und/oder aktiver Bestandteil dieser Kaskade ist. Hierzu gehören Wirkstoffe, welche die Gerinnungskaskade aktivieren, beispielsweise Thrombin [MacGillivray et al., Ann. N.Y. Acad. Sci. 485, 73 (1986)], Thrombin, mutiert im Bereich der Spaltstelle Arg-Thr (Aminosäureposition 327/328), Faktor Va [Cripe et al., Biochem. 31, 3777 (1992), Jenny et al., PNAS USA 84, 4846 (1987)], Faktor VIIa [O'Hara et al., PNAS USA 84, 5158 (1987)], Factor IXa [(Yoshitake et al., Biochem. 24, 3736 (1985)], Faktor Xa [Messier et al., Gene 99, 291 (1991)] oder Tissue factor und gerinnungsaktive Fragmente von ihm, [Morrissey et al., Cell 50, 29 (1987); Scarpati et al., Biochem. 26, 5234 (1987); Spicer et al., PNAS USA 84, 5148 (1987); Rehemtulla et al., Thromb. Heamost. 65, 521 (1991)] oder die Gerinnungskaskade inhibieren oder die Fibrinolyse aktivieren, beispielsweise die Plasminogenaktivatorinhibitoren PAI-1 PAI-2, PAI-3, Hirudin, Protein C, Serin-Proteinase-Inhibitoren, wie beispielsweise C-1S Inhibitor, $\alpha$1-Antritrypsin oder Antithrombin III, Tissue Factor Pathway Inhibitor (TFPI), Plasminogenaktivatoren wie Urokinase, Tissue Plasminogenaktivator (tPA) oder Hybride hiervon, oder die Komplementkaskade aktivieren, beispielsweise Cobra venom factor (CVF) oder Teilsequenzen vom CVF, welche dem menschlichen Komplementfaktor C3b fünktionell entsprechen, d.h. welche an den Komplementfaktor B binden können und nach Spaltung durch den Faktor D eine C3 Konvertase darstellen (die DNA-Sequenz für CVF und seiner Teilsequenzen wurden von Fritzinger et al., Proc. Natl. Acad. Sci. USA 91, 12775 (1994) beschrieben), der menschliche Komplementfaktor C3b (die DNA-Sequenz für C3 und seiner Teilsequenzen wurde von De Bruijn et al., Proc. Natl. Acad. Sci. USA 82, 708 (1985) publiziert), Spaltprodukte des menschlichen Komplementfaktors C3, welche funktionell und strukturell dem CVF ähneln (derartige Spaltprodukte wurden von O'Keefe et al., J. Biol. Chem. 263, 12690 (1988) beschrieben) oder das Kinin-, Komplement- und/oder Gerinnungssystem aktivieren, beispielsweise der aktivierte Hagemann Faktor (F XIIa) [Shibuya et al., Biochem. Biophys. Acta 1206, 63 (1994), Que et al., Biochem. 25, 1525 (1986), Tripodi et al., Nucl. Acid Res. 14, 3146 (1986)] oder Kallikrein [Chen et al., Biochem. J. 307, 481 (1995), Fukushima et al., Biochem. 24, 8037 (1985)],.

Der Wirkstoff (Protein B) kann auch ein zytostatisches, zytotoxisches oder entzündungserregendes Protein sein, wie beispielsweise Perforin, Granzym, Cytokine, wie beispielsweise IL-1, IL-2, TL-4, IL-12, IL-3, IL-5, human leukemia inhibitory factor (LIF), IL-7, IL-11, IL-13, GM-CSF, G-CSFb oder M-CSF, Interferone, wie beispielsweise IFN$\alpha$, IFN$\beta$ oder IFN$\gamma$, TNF, wie beispielsweise TNF$\alpha$ oder TNF$\beta$ Oncostatin M, Sphingomyelinase [Jarvis et al., PNAS USA 91, 73 (1994)], Magainin und Magaininderivate [Cruciani et al., PNAS USA 88, 3792 (1991); Jacob et al., Ciba Found. Symp. 186, 197 (1994); Peck-Miller et al., Cancer Chemother. Pharmac. 32, 109 (1993)], Chemokine, wie beispielsweise RANTES (MCP-2), monocyte chemotactic and activating factor (MCAF), IL-8, macrophage inflammatory protein-1 (MIP-1$\alpha$, -$\beta$) oder neutrophil activating protein-2 (NAP-2).

Der Wirkstoff (Protein B) kann des weiteren ein antiangiogenetisches Protein sein, wie beispielsweise Angiostatin, Interferone, wie beispielsweise IFN$\alpha$, IFN$\beta$ oder IFN$\gamma$, Platelet factor 4, IL-12, TIMP-1, TIMP-2, TIMP-3.

Der Wirkstoff (Protein B) kann auch ein Enzym sein, welches eine inaktive Vorstufe von einer pharmakologischen Wirksubstanz, z. B. eines Zytostatikums in die aktive Wirksubstanz überführen kann. Hierzu gehören beispielsweise bakterielle Nitroreduktase, bakterielle $\beta$-Glucuronidase, pflanzliche $\beta$-Glucuronidase aus Secale cereale, humane $\beta$-Glucuronidase, humane Carboxypeptidase (CB), z.B. CB-A der Mastzelle, CB-B des Pankreas oder bakterielle Carboxypeptidase, bakterielle $\beta$-Laktamase, bakterielle Cytosinedeaminase, humane Catalase bzw. Peroxidase, Phosphatase, im besonderen humane alkalische Phosphatase oder humane saure Prostataphosphatase, Typ 5 saure Phosphatase, Oxidase, im besonderen humane Lysyloxidase oder humane saure D-Aminooxidase, Peroxidase, im besonderen humane Gluthationperoxidase, humane eosinophile Peroxidase oder humane Schilddrüsenperoxidase.

Der Wirkstoff (Protein B) kann des weiteren ein Protein sein, welches das Immunsystem beeinflusst, beispielsweise ein Protein mit antiallergischer Wirkung, wie IFN$\beta$, IFN$\gamma$, IL-10, lösliche IL-4-Rezeptoren, IL-12 oder TGF$\beta$, oder ein Protein, welches die Abstoßung von transplantierten Organen verhindern kann, wie beispielsweise IL-10, TGF$\beta$, lösliche IL-1-Rezeptoren, lösliche IL-2-Rezeptoren, IL-2-Rezeptorantagonisten oder lösliche IL-6-Rezeptoren, oder ein Protein für die Therapie von Antikörper-mediierten Autoimmunerkrankungen, beispielsweise TGF$\beta$, IFN$\alpha$, IFN$\beta$, IFN$\gamma$, IL-12, lösliche IL-4-Rezeptoren oder lösliche IL-6-Rezeptoren, oder ein Protein für die Therapie von Zell-mediierten Autoimmunerkrankungen, beispielsweise IL-6, IL-9, IL-10, IL-13, TNF$\alpha$, IL-4 oder TNF$\beta$, oder ein Protein für die Therapie der Arthritis. Gemäß der vorliegenden Erfindung können auch Strukturgene ausgewählt werden, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert. Hierzu gehören z.B. IL-1-Rezeptorantagonisten (IL-1-RA), da IL-1-RA die Bindung von IL-1$\alpha$, -$\beta$ inhibiert, löslicher IL-1-Rezeptor, da löslicher IL-1-Rezeptor IL-1 bindet und inaktiviert, IL-6, da IL-6 die Sekretion von TIMP und Superoxiden erhöht und die Sekretion von IL-1 und TNF$\alpha$ durch Synovialzellen und Chondrozyten vermindert, löslicher TNF-Rezeptor, da löslicher TNF-Rezeptor TNF bindet und aktiviert, IL-4, da IL-4 die Bildung und Sekretion von IL-1, TNF$\alpha$ und MMP inhibiert, IL-10, da IL-10 die Bildung und Sekretion von IL-1, TNF$\alpha$ und MMP inhibiert und die Sekretion von TIMP erhöht, Insulin-like growth factor (IGF-1), da IGF-1 die Synthese von extrazellulärer Matrix stimuliert, TGF$\beta$, im speziellen TGF$\beta$1 und TGF$\beta$2, da TGF$\beta$ die Synthese von extrazellulärer Matrix Superoxiddismutase stimuliert, oder TIMP (Tissue Inhibitors of Metalloproteinases) im speziellen TIMP-1, TIMP-2 oder TIMP-3.

Der Wirkstoff (Protein B) kann auch ein Protein zur Behebung von Schäden des Nervensystems, beispielsweise ein Wachstumsfaktor, wie FGF, Nerve growth factor (NGF), Brain-derived neurotrophic factor (BDNF), Neurotrophin-3 (NT-3), Neurotrophin-4 (NT-4) oder Ciliary neurotrophic factor (CNTF), oder ein Cytokin oder ein Cytokin-Inhibitor, welcher die neurotoxische Wirkung von TNFα inhibieren oder neutralisieren kann, zum Beispiel TGFβ, lösliche TNF-Rezeptoren, IL-10, da IL-10 die Bildung von IFNγ, TNFα, IL-2 und IL-4 inhibiert, lösliche IL-1-Rezeptoren, wie IL-1-Rezeptor I oder IL-1-Rezeptor II, da lösliche IL-1-Rezeptoren die Aktivität von IL-1 neutralisieren, IL-1-Rezeptor-Antagonist oder lösliche IL-6-Rezeptoren.

Der Wirkstoff (Protein B) kann des weiteren ein Protein sein, welches die Angiogenese stimuliert, wie beispielsweise VEGF oder FGF.

Der Wirkstoff (Protein B) kann des weiteren ein Protein sein, welches den Blutdruck senkt, wie beispielsweise Kallikrein oder Endothelzell "nitric oxide synthase".

Der Wirkstoff (Protein B) kann des weiteren ein Protein für die Therapie von chronischen Infektionserkrankungen sein, beispielsweise ein Protein, welches zytostatische oder zytotoxische Wirkungen aufweist, oder ein Enzym, welches eine Vorstufe einer antiviralen oder zytotoxischen Substanz in die aktive Substanz spaltet, oder ein antiviral wirksames Cytokin oder ein antiviral wirksamer Wachstumsfaktor. Hierzu zählen beispielsweise IFNα, IFNβ, IFNγ, TNFβ, TNFα, IL-1 oder TGFβ.

Ein weiterer Gegenstand der vorliegenden Erfindung ist ein Nukleinsäurekonstrukt, bei dem eine Kombination von zwei gleichen oder zwei unterschiedlichen DNA-Sequenzen, die für gleiche oder unterschiedliche Wirkstoffe (Protein B) [Komponente b) und b")] kodieren, vorliegt.

Zur Expression beider DNA-Sequenzen ist vorzugsweise die cDNA einer "internal ribosome entry site" (IRES) als regulatorisches Element zwischen beiden Strukturen geschaltet. Eine "internal ribosome entry site" ermöglicht die Expression zweier über eine IRES miteinander verbundener DNA-Sequenzen. Derartige IRES wurden beispielsweise von Montford und Smith TIG 11, 179 (1995); Kaufman et al., Nucl. Acids Res. 19, 4485 (1991); Morgan et al., Nucl. Acids Res. 20, 1293 (1992); Dirks et al., Gene 128, 247 (1993); Pelletier und Sonenberg, Nature 334, 320 (1988) und Sugitomo et al., BioTechn. 12, 694 (1994) beschrieben. So kann beispielsweise die cDNA der IRES-Sequenz des Poliovirus (Position ≤ 140 bis ≥ 630 des 5' UTR [Pelletier und Sonenberg, Nature 334, 320 (1988)], zur Verknüpfung der DNA der Komponente c) mit der DNA der Komponente d) verwendet werden.

### Nukleinsäuresequenzen [Komponente c)], die für die durch Proteasen spaltbare Teilstruktur C kodieren:

Gemäß der vorliegenden Erfindung beinhaltet die Teilstruktur C eine Aminosäuresequenz, welche durch Proteasen, die in Tumoren oder von Tumorzellen oder von Entzündungszellen gebildet werden, gespalten wird. Die Nukleinsäuresequenz für diese Teilstruktur C ist beispielsweise in die Nukleinsäuresequenz der natürlicherweise vorliegenden Vorstufe (Protein BSD, wobei S die natürlicherweise vorliegende Spaltsequenz darstellt) des jeweiligen Wirkstoffes (Protein B) anstelle der Spaltsequenz S eingefügt, so daß durch diese rekombinierte Nukleinsäure das Protein BCD bzw. B'BCD exprimiert wird.

Je nach der im Tumor oder in der Entzündung vorwiegend sezernierten Protease wird die Nukleinsäuresequenz, die für die Teilstruktur C kodiert, ausgewählt.

Für folgende Enzyme können beispielsweise folgende Teilstrukturen C eingesetzt werden [Barrett et al., Mammalian Proteases, Academic Press, London (1980), Panchal et al., Nature Biotechnol. 14, 852 (1996); Pigott et al., Ayad et al., The extracellular Matrix, Academic Press (1994); Yoshida et al., Int. J. Cancer 63, 863 (1995), Petersen et al., J. Biol. Chem. 265, 6104 (1990); Cramer et al., J. Urology 156, 526 (1995); Forsgen et al., FEBS Lett. 213, 254 (1987) Zhang et al. Chin. Chem. 41, 1567, 1995)]:

| Enzym | | | | | | | Teilstruktur C Spaltung | |
|---|---|---|---|---|---|---|---|---|
| | S6 | S5 | S4 | S3 | S2 | S1 | S-1 | (S-2) |
| Plasminogenaktivator | | | Cys | Pro | Gly | Arg | Val (Ile) | (Val) |
| | | | | Gln | Gly | Arg | | |
| | | | | Gly | Gly | Arg | | |
| | | | Pro | Arg | Phe | Lys | | |
| | | | | Gly | Lys | Arg | | |

(fortgesetzt)

| Enzym | | S6 | S5 | S4 | S3 | S2 | S1 | Teilstruktur C Spaltung | |
|---|---|---|---|---|---|---|---|---|---|
| | | | | | | | | S-1 | (S-2) |
| Prostatspezifisches Antigen | | | | Pro | Arg | Phe | Lys | Ile | (Ile) (Val) |
| | | | | | Arg | Pro | Tyr | | |
| | | | | Arg | Arg | Phe | Phe | Leu (Ile) | (His) |
| | | | | | | | Tyr | Ile | (Val) |
| | | Ser | Phe | Ser | Ile | Gln | Tyr | Ile | Val |
| | | Gly | Ser | Gln | Gln | Leu | Leu | Ile | Val |
| | | Gly | Ile | Ser | Ser | Gln | Tyr | Ile | Val |
| | | | | | | | | | Val |
| Cathepsine | | | | Pro | Arg | Phe | Lys | Ile | Ile (Val) |
| | | | | | | | Tyr | | |
| | | | | | Lys | Ser | Arg | Met (Ile) | |
| | | | | | Lys | Met | Arg | Arg (Ile) | |
| | | | | | Ile | Arg | Arg | Arg (Ile) | |
| | | | | | Arg | Ala | Arg | Leu (Ile) | |
| | | | | | Gln | Ala | Arg | Phe (Ile) | |
| | | | | | Lys | Leu | Arg | Leu (Ile) | |
| | | | | | | Lys | Arg | Val (Ile) | |
| | | | | | | | Lys | | |
| | | | | | | Phe | Arg | | |
| Stromelysine | | | | Gly | Gly | Gly | Ala | Gln | (Leu) |
| | | | | Gln | Leu | Gly | Val | Met | (Gln) |
| | | | | Ala | Ala | Ala | Ser | Leu | (Lys) |
| | | | | Val | Ala | Val | Ser | Ala | (Lys) |
| | | | | Leu | Ala | Ala | Asn | Leu | (Arg) |
| Collagenase | I | | | Gly | Pro | Gln | Gly | Ile | (Ala) |
| | | | | Gly | Pro | Gln | Gly | Leu | (Leu) |
| | II | | | Gly | Pro | Gln | Gly | Leu | (Ala) |
| | III | | | Gly | Ile | Ala | Gly | Ile | (Thr) |
| | VIII | | | Gly | Leu | Pro | Gly | Ile | (Gly) |
| | | | | Gly | Phe | Pro | Gly | Ile | (Gly) |
| | XI | | | Gly | Pro | Ala | Gly | Ile | (Ser) |
| | | | | Gly | Pro | Ala | Gly | Ile | (Ala) |
| Plasminogen | | | | Ser | Gly | Thr | Glu | Ile | (Val) |

Die Definitionen der Aminosäurenpositionen (S1-S6 und S-1, S-2) erfolgte nach Schechter und Berger, Biochem. Biophys. Res. Comm. <u>27</u>, 157 (1967).

13

#### Nukleinsäuresequenzen [Komponente d)], die für die Teilstruktur D kodieren:

Gemäß der vorliegenden Erfindung kodiert die Nukleinsäuresequenz [Komponente d)] für ein Peptid (Teilstruktur D), welches über die Teilstruktur C an den Wirkstoff (Teilstruktur B) bindet und diesen durch diese Bindung inaktiviert.

Bevorzugt werden für die Teilstruktur D solche Nukleinsäuresequenzen verwendet, welche in den natürlich vorkommenden Vorstufen (Protein BSD) für die Teilstruktur D kodieren, wobei im Protein BSD die Teilstruktur S die natürliche Spaltsequenz darstellt.

Die Strukturen der natürlich vorkommenden Vorstufen von Wirkstoffen (Protein B) wurden bereits übersichtlich dargestellt, so beispielsweise für Gerinnungsfaktoren, Komplementfaktoren und Kallikrein [Bartett et al., Mammalian Proteases, Academic Press, London (1980)], für Interleukine, Chemokine und Wachstumsfaktoren [Callard et al., The Cytokine Facts Book, Academic Press (1994)], für Tissue Inhibitor of Metalloproteinases (TIMPs) [Denhardt et al., Pharmac. Ther. 59, 329 (1993)].

Bei der Auswahl von Wirkstoffen, welche keine natürlich vorkommenden Vorstufen besitzen, und bei xenogenen Wirkstoffen sind als Komponente d) Nukleinsäuresequenzen, die für beliebige Peptide kodieren, zu verwenden, bevorzugt jedoch Nukleinsäuresequenzen, die für solche Teilstrukturen D kodieren, welche in den Vorstufen menschlicher Wirkstoffe natürlicherweise vorkommen.

Zur Erleichterung der Sekretion des von der erfindungsgemäßen Nukleinsäuresequenz exprimierten Proteins BCD bzw. B'BCD kann die ggf. in der DNA-Sequenz der Komponente b) enthaltene homologe Signalsequenz durch eine heterologe, die extrazelluläre Ausschleusung verbessernde Signalsequenz, ersetzt werden. So kann beispielsweise die Signalsequenz für das Immunglobulin [DNA Position $\leq$ 63 bis $\geq$ 107; Riechmann et al., Nature 332, 323 (1988)] oder die Signalsequenz für das CEA [DNA Position $\leq$ 33 bis $\geq$ 134, Schrewe et al., Mol. Cell Biol. 10, 2738 (1990); Berling et al., Cancer Res. 50, 5634 (1990)] oder die Signalsequenz des humanen Respiratory Syncytial Virus Glycoproteins [cDNA der Aminosäuren $\leq$ 38 bis $\geq$ 50 oder 48 bis 65; Lichtenstein et al., J. Gen. Virol. 77, 109 (1996)] eingefügt werden.

Des weiteren kann zur Verstärkung der Translation am 3' Ende der Promotorsequenz und unmittelbar am 5' Ende des Startsignals (ATG) der Signalsequenz die Nukleotidsequenz GCCACC oder GCCGCC [Kozak, J. Cell Biol. 108, 299 (1989)] eingefügt werden.

#### Herstellung der erfindungsgemäßen Nukleinsäurekonstrukte

Die beschriebenen erfindungsgemäßen Nukleinsäurekonstrukte werden dadurch hergestellt, daß die einzelnen Komponenten gemäß molekularbiologischen Standardverfahren miteinander verknüpft werden.

#### Anwendungen:

Das erfindungsgemäße Nukleinsäurekonstrukt eignet sich besonders vorteilhaft zur Behandlung von Erkrankungen, welche mit einer erhöhten lokalen Bildung von Proteasen einhergehen, so beispielsweise von Tumorerkrankungen, Leukämien, Allergien, Autoimmunerkrankungen, Infektionen, Entzündungen, Abstoßungsreaktionen von Transplantaten, Thrombosen und Gefäßverschlüssen und anderen Störungen des Blutgerinnungs- und Blutkreislaufes, Verletzungen von Geweben, einschließlich von Verletzungen des zentralen Nervensystems und Schäden des Nervensystems. Die Verabreichung erfolgt lokal, z.B. auf die Haut, nasal, oral, gastrointestinal, intrabronchial, intravesikal, intravaginal, intrauterin, subkutan, intramuskulär, periartikulär, intraartikulär, in den Liquor, in das Hirngewebe, in das Rückenmark, in Wunden, intraperitoneal oder intrapleural, oder systemisch, z.B. intravenös, intraarteriell, intraportal oder intracardial.

Im allgemeinen enthält das Heilmittel gegebenenfalls neben den üblichen Zusatz- und Hilfsstoffen entweder das erfindungsgemäße Nukleinsäurekonstrukt oder eine Zelle, die das erfindungsgemäße Nukleinsäurekonstrukt exprimieren kann. Das Heilmittel kann zur Prophylaxe oder Therapie einer Erkrankung, wie oben bereits näher beschrieben, verabreicht werden.

Die Herstellung der genannten Zelle erfolgt beispielsweise durch Transformation bzw. Transfektion der Zelle mit dem erfindungsgemäßen Nukleinsäurekonstrukt nach den dem Fachmann bekannten Verfahren.

Beispiele geeigneter Zellen sind Endothelzellen, Lymphozyten, Makrophagen, Gliazellen, Fibroblasten, Leberzellen, Nierenzellen, Muskelzellen, Zellen des Knochen- oder Knorpelgewebes, Synovialzellen, Peritonealzellen, Hautzellen, Epithelzellen, Leukämiezellen und/oder Tumorzellen.

Die erfindungsgemäßen Zellen eignen sich auch zur Herstellung des von dem erfindungsgemäßen Nukleinsäurekonstrukt kodierten Proteins, welches direkt als Heilmittel verwendet werden kann.

Weitere Gegenstände der vorliegenden Erfindung sind daher die Verwendung des erfindungsgemäßen Nukleinsäurekonstruktes zur Herstellung einer gentechnisch veränderten Zelle, wobei das Nukleinsäurekonstrukt in die Zelle eingebracht wird, die Verwendung des erfindinngsgemäßen Nukleinsäurekonstruktes zur Herstellung eines von dem Nukleinsäurekonstrukt kodierten Proteins, wobei das Nukleinsäurekonstrukt in einer geeigneten Zelle zur Expression gebracht und das gebildete Protein isoliert wird, sowie eine Zelle enthaltend das erfindungsgemäße Nukleinsäurekonstrukt. Bevorzugte Zellen sind die oben beschriebenen Zellen.

Je nach Art und Ort der Erkrankung und der zu transduzierenden Zielzelle kann beispielsweise aus den bereits aufgeführten Beispielen für Promotorsequenzen und Strukturgenen (für das Protein BCD bzw. B'BCD) folgende Auswahl getroffen werden:

Therapie von Tumoren

Promotoren [Komponente a)]:
Endothelzell-spezifisch und Zellzyklus-spezifisch oder zellunspezifisch oder Muskelzell-spezifisch und Zellzyklus-spezifisch oder Tumorzell-spezifisch (solide Tumoren, Leukämien)

Liganden für folgende Zielzellen [Komponente b')]:
proliferierende Endothelzellen oder der Endothelzelle benachbarte Stromazellen und Muskelzellen oder Tumorzellen oder Leukämiezellen.

Strukturgene [Komponente b)c)d)]:
für Gerinnung induzierende Faktoren, für Komplementfaktoren, für Angiogeneseinhibitoren, für zytostatische und zytotoxische Proteine, für Induktoren von Entzündungen oder für Enzyme für die Aktivierung von Vorstufen von Zytostatika, zum Beispiel für Enzyme, welche inaktive Vorsubstanzen (Prodrugs) in aktive Zytostatika (Drugs) spalten.

Therapie von Autoimmunerkrankungen und Entzündungen:

Promotoren [Komponente a)]:
Endothelzell-spezifisch und Zellzyklus-spezifisch, oder Makrophagen- und/oder Lymphozyten-spezifisch und/oder Zellzyklus-spezifisch oder Synovialzell-spezifisch und/oder Zellzyklus-spezifisch.

Liganden für folgende Zielzellen [Komponente b')]:
proliferierende Endothelzellen, Makrophagen und/oder Lymphozyten oder Synovialzellen.

Strukturgene [Komponente b)c)d)]:
für die Therapie von Antikörper-mediierten Autoimmunerkrankungen, für Inhibitoren der Zellproliferation, zytostatische oder zytotoxische Proteine, Enzyme für die Aktivierung von Vorstufen von Zytostratika oder für die Therapie von Arthritis.

Therapie von Schäden des Nervensystems:

Promotoren [Komponente a)]:
Gliazell-spezifisch, Endothelzell-spezifisch und Zellzyklus-spezifisch oder unspezifisch und Zellzyklus-spezifisch.

Liganden für folgende Zielzellen [Komponente b')]:
Gliazellen oder proliferierende Endothelzellen

Strukturgene [Komponente b)c)d)]:
für neuronale Wachstumsfaktoren, zum Beispiel für Cytokine und Cytokin-Inhibitoren, welche die neurotoxische Wirkung von TNF$\alpha$ inhibieren oder neutralisieren.

Therapie von Störungen des Blutgerinnungs- und Blutkreislaufsystems:

Promotoren [Komponente a)]:
zellunspezifisch, zellunspezifisch und Zellzyklus-spezifisch oder spezifisch für Endothelzellen, glatte Muskelzellen oder Makrophagen, oder spezifisch für Endothelzellen, glatte Muskelzellen oder Makrophagen und Zellzyklus-spezifisch.

Liganden für folgende Zielzellen [Komponente b')]:
Endothelzellen, proliferierende Endothelzellen oder somatische Zellen in Nachbarschaft von Endothelzellen und glatte Muskelzellen oder Makrophagen.

Stukturgene [Komponente b)c)d)]:
für die Inhibition der Gerinnung oder für die Förderung der Fibrinolyse, für Angiogenesefaktoren, für Blutdruck senkende Peptide, für ein antiproliferatives, zytostatisches oder zytotoxisches Protein oder für ein Enzym zur Aufspaltung von Vorstufen von Zytostatika in Zytostatika zur Inhibition der Proliferation von glatten Muskelzellen nach Verletzungen der Endothelschicht oder für Blutplasmaproteine, wie C1-Inaktivator, Serum Cholinesterase oder $\alpha$1-Antritrypsin.

Therapie von chronischen Infektionserkrankungen

Promotoren [Komponente a)]:
virusspezifisch, zellspezifisch oder virusspezifisch oder zellspezifisch und zellzyklusspezifisch.

Liganden für folgende Zielzelle [Komponente b')]:
Leberzelle, Lymphozyt und/oder Makrophage, Epithelzelle oder Endothelzelle.

Strukturgene [Komponenten b)c)d)]:
für ein Protein, welches zytostatische oder zytotoxische Wirkungen aufweist, ein Enzym, welches eine Vorstufe einer antiviralen oder zytotoxischen Substanz in die aktive Substanz spaltet, oder für antivirale Proteine, wie beispielsweise antiviral wirksame Cytokine und Wachstumsfaktoren.

Die Erfindung wird anhand der folgenden Beispiele und Figuren näher erläutert, ohne sie darauf zu beschränken:

**Beschreibung der Figuren**

Figur 1:     Schematische Darstellung eines erfindungsgemäßen Nukleinsäurekonstrukts enthaltend die Komponenten a), b), c) und d).

Figur 2:     Schematische Darstellung eines erfindungsgemäßen Nukleinsäurekonstrukts erweitert um die Komponente b').

Figur 3:     Schematische Darstellung eines Nukleinsäurekonstrukts für den durch PSA aktivierbaren Faktor X.

**Beispiel**

1. Herstellung eines Nukleinsäurekonstruktes kodierend für den Prostataspezifischen Antigen (PSA)-aktivierbaren FX

Es wird ein Therapeutikum für die Behandlung von Metastasen des Prostatakarzinoms hergestellt. Trotz chirurgischer Entfernung einer zum Karzinom erkrankten Prostata treten häufig Metastasen des Prostatakarzinoms auf, die derzeit noch weitgehend unheilbar sind und zum Tod des Patienten führen. Derartige Prostatakarzinommetastasen induzieren Angiogenese. Des weiteren sezernieren Prostatakarzinommetastasen ein gewebespezifisches Enzym, das Prostata-spezifische Antigen (PSA). Entsprechend der Erfindung wird ein Nukleinsäurekonstrukt hergestellt, welches eingeführt in proliferierende Endothelzellen zur Expression eines modifizierten Gerinnungsfaktors FX führt. Die Modifikation besteht darin, daß im Gen für den natürlichen FX die Nukleotidsequenz für die natürliche Spaltstelle, deren Spaltung zum gerinnungsaktiven FXa führt gegen eine Nukleotidsequenz kodierend für eine PSA-spezifische Spaltstelle ausgetauscht ist. Hierdurch ist das von Prostatakarzinommetastasen sekretierte PSA in der Lage, das von proliferierenden Endothelzellen in der Nachbarschaft der Metastasen sekretierte modifizierte FX spezifisch zu aktivieren und hierdurch die Gerinnung einzuleiten, welche zur Unterbrechung der Blutzufuhr der Metastase und damit zu deren Nekrose führt.

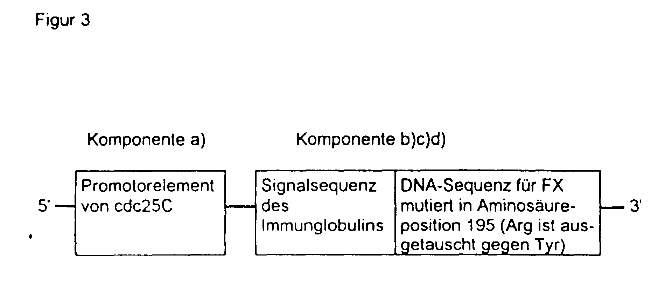

Das Nukleinsäurekonstrukt für den durch PSA aktivierbaren FX wird nach einem Schema hergestellt, welches in Figur 3 dargestellt ist.

Die DNA-Sequenzen der Einzelkomponenten werden in Richtung 5' zu 3' wie folgt zusammengefügt:

Die Komponente a), die die Promotorsequenz des cdc25C Genes [Nukleotide: -290 bis +121; Lucibello et al., EMBO J. 14, 132 (1995); Zwicker et al., Nucl. Acids Res 23, 3822 (1995); EMBO J. 14, 4514 (1995)), die Sequenz GCCACC (Kozak, J. Cell Biol. 108, 229 (1989)) und die cDNA für das Signalpeptid des Immunglobulins [Nukleotidsequenz $\leq$ 63 bis $\geq$ 107; Riechmann et al., Nature 332, 323 (1988)) enthält, wird an die Komponente b)c)d), die die cDNA des humanen FX (Nukleotidsequenz 1 bis $\geq$ 1468) [Messier et al., Gene 99, 291 (1991)] mit Mutation in Aminosäure 194 von Arg nach Tyr enthält, fusioniert.

Die Verknüpfung der einzelnen Bestandteile des Konstruktes erfolgt über geeignete Restriktionsstellen, die über PCR-Amplifikation an den Termini der verschiedenen Elemente angeführt werden. Die Verknüpfung erfolgt mit Hilfe von den dem Fachmann bekannten, für die Restriktionsstellen spezifischen Enzymen und DNA-Ligasen. Diese Enzyme sind käuflich zu erwerben.

Das so hergestellte Nukleotidkonstrukt wird in pUC 18/19 oder Bluescript-abgeleiteten Plasmidvektoren einkloniert.

## 2. Expression in humanen embryonalen Nierenzellen

Mit dem beschriebenen Plasmid werden in Kultur gehaltene proliferierende menschliche embryonale Nierenzellen [HEK 293; Racchi et al., J. Biol. Chem. 268, 5735 (1993)] mit der dem Fachmann bekannten Methode [Graham und van der Eb, Virol. 52, 456 (1973)] transfiziert.

Aus dem Überstand von ca. $10^7$ transfizierten HEK 293-Zellen wird der mutierte Faktor X angereinigt [Watzke et al., J. Clin. Invest. 88, 1685 (1991)] und in einem Gerinnungstest für Faktor X mit und ohne Zusatz von PSA geprüft. Gereinigtes PSA wird von Chemicon (Temecula, CA, USA) bezogen.

In diesem Test wird der Koagulationsdefekt von menschlichem FX Mangelplasma durch funktionell aktives FXa kompensiert.

Als Positivkontrolle wird nicht mutierter (Wildtyp) FX (aktiviert durch Russel's viper venom) eingesetzt. Neben dem Testansatz ohne PSA dient als Negativkontrolle eine Mockpräparation aus dem Überstand nichttransfizierter HEK 293-Zellen.

Die Gerinnungsaktivität des mutierten FX wird mittels Rekalzifizierungszeit (Seitz R et. al, Int. J. Cancer 53:514-520, 1993) gemessen. 100 µl Fx-Mangelplasma (Behringwerke, Marburg) werden mit 100 µl der FX-Präparation aus dem Zellüberstand für 120 sec bei 37°C inkubiert. Die FX-Präparation enthält PSA als Aktivator. Für die Negativkontrolle wird kein PSA zugefügt. Als Positivkontrolle wird FX (Wildtyp) und Russel Viper Venom (RVV) eingesetzt. Die Gerinnungsreaktion wird durch Zugabe von 100 µl 0.02 M $CaCl_2$ gestärkt und in einem Koagulometer bestimmt.

Folgende Ergebnisse werden erzielt:

Die Negativkontrollen ohne Aktivierung der Gerinnung ergeben eine Gerinnungszeit von ca. 200 sec. Im Gegensatz hierzu lassen sich mit aktiviertem FX (mutiertes FX und PSA bzw. Wildtyp FX und RVV) signifikant verkürzte Gerinnungszeiten von 50 sec erreichen.

Daraus kann geschlossen werden, daß die transduzierten HEK 293-Zellen mutierten FX exprimieren, welcher unter Zusatz von PSA den Koagulationsdefekt von FX Mangelplasma kompensiert.

## 3. Expression in humanen Endothelzellen

Mit dem beschriebenen Plasmid werden in Kultur gehaltene menschliche Nabelschnurendothelzellen mit der dem Fachmann bekannten Methode (Lucibello et al., EMBO J. 14, 132 (1995)) transfiziert.

Zur Überprüfung der Zellzyklusspezifität werden Endothelzellen durch Entzug von Methionin über 48 Stunden in G0/G1 synchronisiert. Der DNA-Gehalt der Zellen wird nach Anfärben mit Hoechst 33258 (Hoechst AG, Frankfurt) im Fluoreszenzaktivierungs-Cell Sorter bestimmt (Lucibello et al., EMBO J. 14, 132 (1995)).

Die Expression des Nukleinsäurekonstruktes wird im Überstand der Endothelzellen analog der Untersuchung an HEK 293-Zellen überprüft.

Folgende Ergebnisse werden erzielt:

Das exprimierte Protein transfizierter Endothelzellen führt zur Kompensation des Koagulationsdefektes von FX Mangelplasma im Gegensatz zu Mockpräparationen aus dem Überstand nichttransfizierter Endothelzellen.

Im Überstand von proliferierenden, transduzierten Endothelzellen (DNA > 2S) ist eine deutlich höhere Konzentration mutierten FX nachzuweisen als im Überstand von in G0/G1 synchronisierten Endothelzellen (DNA = 2S).

Somit führt das beschriebene Nukleinsäurekonstrukt zu einer zellzyklusabhängigen Expression des Genes für den mutierten FX in Endothelzellen und dieses mutierte FX kann durch PSA aktiviert werden, so daß es im FX Mangelplasma eine Gerinnung bewirkt.

## Patentansprüche

1. Nukleinsäurekonstrukt, zur Expression einer Wirksubstanz, die durch ein von Säugerzellen freigesetztes Enzym aktiviert wird, dadurch gekennzeichnet, daß das Konstrukt folgende Komponenten enthält:

   a) mindestens ein Promotorelement,
   b) mindestens eine DNA-Sequenz, die für einen Wirkstoff (Protein B) kodiert,

c) mindestens eine DNA-Sequenz, die für eine Aminosäuresequenz (Teilstruktur C) kodiert, welche durch ein von einer Säugerzelle freigesetztes Enzym spezifisch spaltbar ist, und

d) mindestens eine DNA-Sequenz, die für ein Peptid oder Protein (Teilstruktur D) kodiert, welches über die spaltbare Aminosäuresequenz (Teilstruktur C) an den Wirkstoff (Protein B) gebunden ist und die Wirksamkeit des Wirkstoffes (Protein B) hemmt.

2. Nukleinsäurekonstrukt nach Anspruch 1, dadurch gekennzeichnet, daß das genannte Enzym eine Protease ist.

3. Nukleinsäurekonstrukt nach Anspruch 1 oder 2, dadurch gekennzeichnet, daß das genannte Enzym ein Prostata-spezifisches Antigen, ein Plasminogenaktivator, ein Cathepsin oder eine Matrix-Metalloproteinase ist.

4. Nukleinsäurekonstrukt nach einem der Ansprüche 1 bis 3, dadurch gekennzeichnet, daß die genannten Säugerzellen Tumorzellen, Leukämiezellen, Endothelzellen, Makrophagen, Lymphozyten, Muskelzellen, Epithelzellen, Gliazellen, Synovialzellen oder virusinfizierte Zellen sind.

5. Nukleinsäurekonstrukt nach einem der Ansprüche 1 bis 4, dadurch gekennzeichnet, daß das Nukleinsäurekonstrukt um die Komponente b') ergänzt ist, die für einen Liganden (Teilstruktur B') kodiert, welcher den Wirkstoff (Protein B) an eine Zielstruktur bindet.

6. Nukleinsäurekonstrukt nach einem der Ansprüche 1 bis 5, dadurch gekennzeichnet, daß das genannte Protein B und die Teilstruktur D Teile der natürlichen Vorstufen von Proteinwirkstoffen sind, wobei die natürliche Spaltsequenz, welche die Teilstrukturen B und D verbindet, durch die Teilstruktur C ersetzt worden ist.

7. Nukleinsäurekonstrukt nach einem der Ansprüche 1 bis 6, dadurch gekennzeichnet, daß die genannte Teilstruktur D die Teilstruktur einer natürlichen Vorstufe eines Proteinwirkstoffes ist.

8. Nukleinsäurekonstrukt nach einem der Ansprüche 1 bis 7, dadurch gekennzeichnet, daß es in ein Plasmid oder einen viralen Vektor eingefügt ist.

9. Nukleinsäurekonstrukt nach einem der Ansprüche 1 bis 8, dadurch gekennzeichnet, daß die Komponente a) eine unspezifisch, eine zellspezifisch, eine virusspezifisch, eine metabolisch, eine zellzyklusspezifisch und/oder eine durch Tetrazyklin aktivierbare Promotorsequenz darstellt.

10. Nukleinsäurekonstrukt nach einem der Ansprüche 1 bis 9, dadurch gekennzeichnet, daß die Komponente a) eine Kombination von mindestens zwei gleichen oder unterschiedlichen Promotorsequenzen darstellt.

11. Nukleinsäurekonstrukt nach einem der Ansprüche 9 oder 10, dadurch gekennzeichnet, daß die Komponente a) bevorzugt in Endothelzellen, in Zellen benachbart zu aktivierten Endothelzellen, in Muskelzellen, in Leukämiezellen, in Tumorzellen, in Gliazellen, in Lymphozyten, in Makrophagen und/oder in Synovialzellen aktiviert wird.

12. Nukleinsäurekonstrukt nach einem der Ansprüche 1 bis 11, dadurch gekennzeichnet, daß der genannte Wirkstoff (Protein B) eine biologische Aktivierungskaskade aktiviert oder inhibiert und/oder aktiver Bestandteil dieser Kaskade ist, vorzugsweise das Gerinnungssystem aktiviert oder inhibiert, die Fibrinolyse aktiviert, das Komplementsystem aktiviert und/oder das Kininsystem aktiviert, oder ein Enzym ist, welches die inaktive Vorstufe einer pharmakologischen Substanz in die aktive Substanz überführt oder selbst eine pharmakologisch aktive Substanz darstellt.

13. Nukleinsäurekonstrukt nach Anspruch 12, dadurch gekennzeichnet, daß der genannte Wirkstoff (Protein B) einen Gerinnungsfaktor darstellt, ausgewählt aus Thrombin, Faktor Va, Faktor VIIa, Faktor IXa, Faktor Xa, TF gerinnungsaktive Fragmente oder Faktor XIIa; Thrombin, mutiert im Bereich der Spaltstelle Arg-Thr (Aminosäureposition 327/328); ein fibrinolytisches Protein ausgewählt aus Urokinase, tPA oder funktionellen Hybriden hiervon; einen Komplementfaktor ausgewählt aus CVF, C3b oder funktionellen Spaltprodukten hiervon; ein antithrombotisches Protein ausgewählt aus Protein C, C-1S-Inhibitor, $\alpha$1-Antitrypsin, Hirudin, AT-III, TFPI, PAI-1, PAI-2 oder PAI-3; ein Kallikrein; ein zytostatisches, zytotoxisches oder entzündungserregendes Protein; ein antiangiogenetisches Protein; ein immunmodulierendes Protein; ein antientzündlich-wirkendes Protein; ein Schäden des Nervensystems behebendes Protein; ein die neurotoxische Wirkung von TNF$\alpha$ inhibierendes oder neutralisierendes Protein; ein die Angiogenese stimulierendes Protein; ein den Blutdruck senkendes Protein; ein antivirales Protein; ein Cytokin; ein Interferon; einen Tumornekrosefaktor; Oncostatin M oder LIF; einen Cytokinrezeptor; den zellexternen Teil eines Cytokinrezeptors; einen Cytokinantagonisten; einen Wachstumsfaktor einen Wachstumsfaktorrezeptor; den zellexternen Teil eines Wachstumsfaktorrezeptors; ein Chemokin; Angiostatin; Platelet factor 4; TIMP-1, - 2 oder - 3; eine Nitroreduktase; eine β-Glucuronidase; eine Carboxypeptidase; eine β-Laktamase; eine Cytosindeaminase; eine Katalase; eine Peroxidase; eine Phosphatase; eine Oxidase; Kallikrein oder eine Endothelzellnitritoxidsynthase.

14. Nukleinsäurekonstrukt nach einem der Ansprüche 1 bis 13, dadurch gekennzeichnet, daß die Komponente b') für einen Liganden (Teilstruktur B') kodiert, welcher an die Oberfläche von Zellen, vorzugsweise an einen Zellmembranrezeptor, an ein Zellmembranantigen, an ein zellmembranständiges Adhäsionsmolekül, an die extrazelluläre Matrix oder Komponenten davon bindet.

15. Nukleinsäurekonstrvkt nach einem der Ansprüche 1 bis 14, dadurch gekennzeichnet, daß der Ligand ein Antikörper oder ein Antikörperfragment ist, der/das spezifisch an ein Zellmembranantigen oder an ein Antigen auf der extrazellulären Matrix bindet, oder ein Peptid oder Protein ist, welches an einen Rezeptor auf der Zellmembran bindet, vorzugsweise ein Wachstumsfaktor, ein Cytokin, ein Interferon, ein Tumornekrosefaktor, ein Chemokin, eine Rezeptor-bindende Teilsequenz dieser Liganden, ein Peptidhormon, Angiotensin, Kinin, Folsäure, ein Adhäsionsmolekül oder die an das korrespondierende Adhäsionsmolekül oder an die extrazelluläre Matrix bindende Teilsequenz des Adhäsionsmoleküls, ein extrazellulärer Teil eines Fc-Rezeptors, ein Glykoprotein eines Virus, einer an diese Zellen bindende Teilsequenz des Glykoproteins, die Transmembrandomäne eines Rezeptors oder eines viralen Glykoproteins oder ein Glykophospholipidanker.

16. Nukleinsäurekonstrukt nach Anspruch 14 oder 15, dadurch gekennzeichnet, daß der Ligand an Endothelzellen, vorzugsweise an aktivierte oder proliferierende Endothelzellen, an Tumorzellen, an Muskelzellen, vorzugsweise glatten Muskelzellen, an Fibroblasten, an Makrophagen, an Lymphozyten, an Leberzellen, an Nierenzellen, an Synovialzellen, an Entzündungszellen, an mit Viren infizierte Zellen, an Bronchialepithelzellen, an Gliazellen oder an Leukämiezellen bindet.

17. Nukleinsäurekonstrukt nach einem der Ansprüche 1 bis 16, dadurch gekennzeichnet, daß das Konstrukt mindestens zwei gleiche oder unterschiedliche Komponenten b)c)d) bzw. b')b)c)d) enthält, welche über eine internal ribosomal entry site (IRES) miteinander verbunden sind.

18. Verfahren zur Herstellung eines Nukleinsäurekonstrukts nach einem der Ansprüche 1 bis 17, dadurch gekennzeichnet, daß die genannten Komponenten miteinander verknüpft werden.

19. Verwendung eines Nukleinsäurekonstrukts nach einem der Ansprüche 1 bis 17 zur Herstellung eines Heilmittels zur lokalen oder systemischen Verabreichung zur Prophylaxe und/oder Therapie von Tumoren, Leukämien, Allergien, Autoimmunerkrankungen, Infektionen, Entzündungen, Abstoßungsreaktionen von Transplantaten, Thrombosen, Gefäßverschlüssen, Störungen des Blutgerinnungs- und Blutkreislaufes, Verletzungen von Geweben und/oder Schäden des Nervensystems.

20. Verwendung eines Nukleinsäurekonstrukts nach einem der Ansprüche 1-17 zur Herstellung einer gentechnisch veränderten Zelle, dadurch gekennzeichnet, daß das genannte Nukleinsäurekonstrukt in eine geeignete Zelle eingebracht wird.

21. Verwendung eines Nukleinsäurekonstrukts nach einem der Ansprüche 1-17 zur Herstellung eines von dem genannten Nukleinsäurekonstrukt kodierten Proteins, dadurch gekennzeichnet, daß das genannte Nukleinsäurekonstrukt in einer geeigneten Zelle zur Expression gebracht und das gebildete Protein isoliert wird.

22. Verwendung nach Anspruch 20 oder 21, dadurch gekennzeichnet, daß die genannte Zelle eine Endothelzelle, ein Lymphozyt, ein Makrophage, eine Gliazelle, ein Fibroblast, eine Leberzelle, eine Nierenzelle, eine Muskelzelle, eine Zelle des Knochen- oder Knorpelgewebes, eine Synovialzelle, eine Peritonealzelle, eine Hautzelle, eine Epithelzelle, eine Leukämiezelle und/oder eine Tumorzelle ist.

23. Zelle enthaltend ein Nukıeinsäurekonstrukt gemäß einem der Ansprüche 1-17.

Figur 1

Strukturgen

| Komponente a) | Komponente b) | Komponente c) | Komponente d) |
|---|---|---|---|
| Promoter-element | DNA-Sequenz kodierend für einen Wirkstoff (Protein B) | DNA-Sequenz kodierend für eine durch Tumor-proteasen spaltbare Aminosäuresequenz (Teilstruktur C) | DNA-Sequenz kodierend für ein Peptid (Teilstruktur D), welches den Wirkstoff (Protein B) inhibiert |

5'

3'

vom Strukturgen kodiertes Protein BCD

EP 0 859 058 A2

Figur 2

Strukturgen

| Komponente a) | Komponente b') | Komponente b) | Komponente c) | Komponente d) |
|---|---|---|---|---|
| Promoter-element | DNA-Sequenz kodierend für einen Liganden (Teilstruktur B') | DNA-Sequenz kodierend für einen Wirkstoff (Protein B) | DNA-Sequenz kodierend für eine durch Tumorproteasen spaltbare Aminosäuresequenz (Teilstruktur C) | DNA-Sequenz kodierend für ein Peptid (Teilstruktur D), welches den Wirkstoff (Protein B) inhibiert |

5'

3'

vom Strukturgen kodiertes Protein B'BCD

EP 0 859 058 A2

Figur 3

EP 0 859 058 A2

Komponente a)          Komponente b)c)d)

5' ── | Promotorelement von cdc25C | ── | Signalsequenz des Immunglobulins | DNA-Sequenz für FX mutiert in Aminosäure-position 195 (Arg ist aus-getauscht gegen Tyr) | ── 3'